# EUROPÄISCHE PATENTSCHRIFT

(11) **EP 1 337 229 B1**
(45) Veröffentlichungstag und Bekanntmachung des Hinweises auf die Patenterteilung: **30.09.2009**
(21) Anmeldenummer: 01990457.2
(22) Anmeldetag: 29.11.2001
(51) Int. Cl.: A61K 8/60, A61Q 5/12, D06M 13/148, D06M 15/03

(54) **IMMOBILISIERUNG VON WIRKSTOFFEN AN FASERN**
IMMOBILISATION OF ACTIVE AGENTS ON FIBRES
IMMOBILISATION D'AGENTS ACTIFS SUR DES FIBRES

(30) Priorität: 01.12.2000 DE 10059749
(43) Veröffentlichungstag der Anmeldung: 27.08.2003
(73) Patentinhaber: Henkel AG & Co. KGaA, 40589 Düsseldorf (DE)
(72) Erfinder: BUSCH, Peter, 40699 Erkrath (DE); GASSENMEIER, Thomas, Otto, 40229 Düsseldorf (DE); NAUMANN, Frank, 40219 Düsseldorf (DE); HUCHEL, Ursula, 50670 Köln (DE); BABIEL, Sabine, 47447 Moers (DE); KLEEN, Astrid, 40699 Erkrath (DE)
(86) Internationale Anmeldenummer: PCT/EP2001/013965
(87) Internationale Veröffentlichungsnummer: WO 2002/043675

(56) Entgegenhaltungen:
- EP-A- 0 788 832
- EP-A- 0 826 661
- EP-A- 0 873 744
- CH-A- 429 027
- GB-A- 575 253
- US-A- 4 036 589
- US-A- 6 146 619
- W. UMBACH: "Kosmetik: Entwicklung, Herstellung u. Anwendung kosmet. Mittel" 1995 , GEORG THIEME VERLAG , SUTTGART; NEW YORK XP002198397 Seite 294 -Seite 303; Abbildung 8.5.1

## Beschreibung

Die Erfindung betrifft ein Verfahren zur Immobilisierung von Wirkstoffen an Fasern vorzugsweise Haaren gemäß Anspruch 1 eine Zusammensetzung gemäß Anspruch 27 umfassend reaktive Wirkstoff-Komponenten und deren Verwendung zur Restrukturierung, Wiederherstellung sowie zur positiven Beeinflussung der Fasereigenschaften wie Volumen, Glanz, Halt, Fülle, Taktilität, Elektrostatik und Resistenz gegenüber Hitze, UV- und IR-Strahlung.

Die Reinigung und Pflege der Haare ist ein wichtiger Bestandteil der menschlichen Körperpflege. Sowohl die Reinigung der Haare mit Shampoos als auch die dekorative Gestaltung der Frisur, beispielsweise durch Färben oder Dauerwellen sind Eingriffe, die die natürliche Struktur und die Eigenschaften der Haare beeinflussen. So können anschließend an eine solche Behandlung beispielsweise die Nass- und Trockenkämmbarkeit, Halt, Fülle, Glanz und Taktilität des Haares unbefriedigend sein. Zusätzlich erfährt das Haar eine Schädigung durch Umwelteinflüsse wie UV-, IR-Strahlung und Wärmeeinwirkung, z. B. durch Fönen des Haares.

Zur Erhaltung und Wiederherstellung der vorgenannten Eigenschaften ist es daher seit langem üblich, die Haare einer speziellen Nachbehandlung zu unterziehen. Dabei werden die Haare, üblicherweise in Form einer Spülung, mit speziellen Wirkstoffen, beispielsweise quaternären Ammoniumsalzen oder speziellen Polymeren, behandelt. Dadurch werden je nach Formulierung Kämmbarkeit, Halt, Fülle, Glanz und Griff der Haare verbessert. Für die temporäre Beeinflussung des Haarvolumens ist der Einsatz von Polyaminoglucosen wie in EP 0412745 und US 5,523,078, von Cellulose wie in JP 04124121 und EP 0823252 sowie Alkylcellulose in WO 97/38673, US 5,534,247 und WO 94/06410 beschrieben. Des weiteren finden auch kleinere Kohlenhydrate wie Glucose in WO 95/00104, Maltose in WO 95/00104 und Lactose in DE 19610458 Anwendung. Bei der Applikation solcher Spülungen verbleibt jedoch nur ein relativ geringer Anteil der Wirkstoffe an seinem Wirkort. Der Großteil wird sofort abgespült oder durch späteres Waschen der Haare oder mechanische Beanspruchung, wie z.B. Kämmen, von der Haaroberfläche abgelöst.

CH 429 027 offenbart ein Verfahren zur Herstellung einer dauerhaften Frisur, bei dem auf das Haar zunächst eine reaktive Komponente (A) mit mindestens zwei Epoxy-Gruppen und anschließend als reaktive Komponente (B) eine Substanz, ausgewählt aus primären und sekundären Di- und Polyaminen, Mercaptoguppen enthaltenden Monoaminen und Kohlenwasserstoffcarbonsäuren, aufgetragen wird, wobei (A) und (B) in einer Additionsreaktion einen Polymerfilm auf dem Haar bilden. Weiterhin ist im Stand der Technik die Bildung oxidativer Haarfarben bekannt, die durch die Zugabe eines Oxidationsmittels zu aromatischen Oxidationsfarbstoffvorprodukten, die mindestens zwei Amino- oder Hydroxygruppen, aber keine Carbonylgruppe aufweisen, initiiert wird. EP 788832 A1 offenbart ein Verfahren zur Haarbehandlung, bei dem auf das Haar in einem einzigen Schritt das zuvor fertig gestellte Endprodukt einer Additionsreaktion aus einer Epoxyverbindung und einem primären oder sekundären Amin, ein Aminoalkohol mit tensidischer und konditionierender Wirkung, aufgetragen wird. EP 788832 A1 offenbart keine in-situ-Reaktion eines primären Amins (A) mit einer Carbonylverbindung (B) zu einer Schiffschen Base direkt auf dem Haar. EP 826 661 A1 offenbart ein Verfahren zur Haarbehandlung, bei dem ein veresterter Wirkstoff auf das Haar aufgetragen wird und dort nach und nach durch hydrolytische Spaltung den Wirkstoff wieder freisetzt, also als Wirkstoffreservoir dient.

Die US 3,619,114 lehrt die dauerhafte Modifizierung keratinöser Substrate durch Copolymerisation mit Vinylmonomeren in Gegenwart radikalischer Katalysatoren und anschließender Behandlung mit wässrigen ammoniakalischen Kupferhydroxydlösungen. Aufgrund der Toxizität der eingesetzten Verbindungen, beschränkt sich die Anwendung jedoch auf nichtbelebte keratinische Fasern. US 2,615,782 lehrt die Modifikation von keratinösen Fasern durch Knüpfung von Disulfid-Brücken zwischen Haar und Wirksubstanz. Eine weitere Methode zur Fixierung von Verbindungen, die der dauerhaften Verformung von Haaren dienen und die insbesondere auf Dauerwellprozesse abzielt, ist der Einsatz sogenannter Bunte-Salz-Derivate, wie in DE 3735086, DE 4109869 und EP 0246151 beschrieben. US 3,415,606 lehrt die Verwendung von Reaktivfarbstoffen, die eine Langzeitwirkung auf dem Haar entfalten. Es ist daher von Interesse, Wirkstoffe vorzugsweise dauerhaft am Haar zu fixieren und zugleich eine Belastung der Umwelt und eine gesundheitliche Beeinträchtigung des Anwenders zu vermeiden.

Die Aufgabe wird erfindungsgemäß gelöst durch ein Mehrkomponenten-System, vorzugsweise bestehend aus wenigstens zwei Komponenten (A) und (B). In einem denkbaren Modell ist ein Mechanismus vorstellbar, bei dem beide Komponenten unabhängig voneinander ins Innere der Fasern oder bei Haaren in die Cuticula penetrieren können um dort miteinander zu reagieren. Die erfindungsgemäße, lang anhaltende, vorzugsweise dauerhafte Wirkung ist möglicherweise darauf zurückzuführen, dass das gebildete, den oder die Wirkstoffe enthaltende Produkt aufgrund seiner Größe die Hohlräume der Fasern nicht mehr verlassen kann.

Dabei soll jedoch keine Aussage Ober den Ort der Immobilisierung gemacht werden. Obwohl die modellhafte Vorstellung existiert, dass sich die immobilisierten Wirkstoffe in den Hohlräumen der Fasern befinden, ist nicht auszuschließen, dass diese auch an der Haaroberfläche adsorbiert sind.

Im einzelnen betrifft die Erfindung ein Verfahren zur Immobilisierung von Wirkstoffen an Fasern.

Zur dauerhaften und positiven Beeinflussung des Volumens, der Naß- und Trockenkämmbarkeit, des Halts, der Feuchtigkeit, der Fülle, des Glanzes, der Festigkeit, der Taktilität, der elektrostatischen Eigenschaften, der erhöhten Resistenz gegen UV- und IR-Strahlung und Wärmeeinwirkung von Fasern, insbesondere von menschlichen und tierischen Haaren, durch Immobilisierung von Wirkstoffen an Fasern, durch deren Behandlung mit einem Mehrkomponenten-System, umfassend
- wenigstens eine reaktive Komponente (A) mit einem Molekulargewicht ≤1000 Dalton, die wenigstens eine funktionelle Gruppe aufweist, die ausgewählt ist aus primären Amino-Gruppen,
- wenigstens eine komplementäre reaktive Komponente (B) mit einem Molekulargewicht ≤1000 Dalton, die wenigstens eine funktionelle Gruppe aufweist, die ausgewählt ist aus Carbonyl-Gruppen,
wobei die Komponenten (A) und (B) miteinander unter Bildung einer Schiffschen Base reagieren und sich wenigstens eine der Komponenten (A) und (B) von einem Wirkstoff ableitet, der ausgewählt ist aus der Gruppe, bestehend aus Substanzen zur positiven Beeinflussung von Volumen, Griff, Halt und Fülle von Fasern, insbesondere Haaren, Pflege-, Restrukturierungs- und Repairwintstoffen. Kohlenhydraten, Aminosäuren, Vitaminen und UV-Filtern, wobei die in den reaktiven Komponenten enthaltenen funktionellen Gruppen durch geeignete Derivatisierung in die Wirkstoffmoleküle eingeführt werden können.

Der Begriff einer komplementären reaktiven Komponente stellt darauf ab, dass die Komponenten die zur Knüpfung der zuvor genannten Bindungen notwendigen Funktionalitäten aufweisen. Beispiele für geeignete Kombinationen sind nachfolgend beschrieben.

Im Rahmen der vorliegenden Erfindung geeignete Fasern sind menschliche und tierische Haare, Wolle, Seide, Cellulosefasern, Kunstfasern, Felle und Pelze, insbesondere jedoch menschliche und tierische Haare.

In der erfindungsgemäßen Ausführungsform weist
. Komponente (A) wenigstens eine primäre Amino-Gruppe auf, während
. Komponente (B) wenigstens eine Carbonyl-Gruppe (Aldehyd- oder Keton-Gruppe) besitzt.

Beide Komponenten reagieren miteinander, gemäß der nachfolgenden Gleichung I, unter Knüpfung einer kovalenten Bindung zu einer Schiffschen Base (A-B).

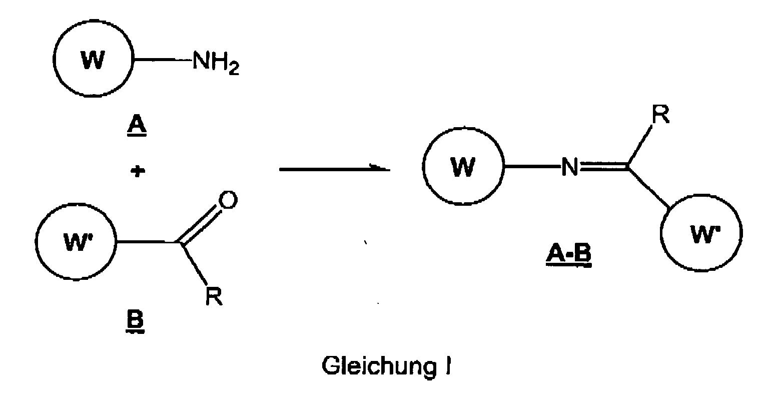

Gemäß der Erfindung leiteten sich entweder einzelne oder mehrere reaktive Komponenten, unabhängig voneinander, von Wirkstoffen, vorzugsweise haarkosmetischen Wirkstoffen (W,W') ab, die ausgewählt sind aus der Gruppe bestehend aus
- Kohlenhydraten,
- Aminosäuren,
- Vitaminen und
- UV-Filtern.

Die reaktiven Komponenten, besitzen ein Molekulargewicht von 160 bis zu 1000 Dalton, vorzugsweise bis zu 550 Dalton.

Die in den reaktiven Komponenten enthaltenen funktionellen Gruppen sind ausgewählt aus primären Amino-Gruppen (-NH₂) und Carbonyl-Gruppen (-(C=O)-) und können, soweit sie nicht schon in den Wirkstoffen an sich enthalten sind, durch geeignete Derivatisierung in die Wirkstoffmolekole eingeführt werden.

In einer erfindungsgemäßen Ausführungsform ist wenigstens eine der reaktiven Komponenten ein Kohlenhydrat oder von einem solchen abgeleitet.

Bevorzugte Kohlenhydrate sind ausgewählt aus der Gruppe bestehend aus Aldohexosen, Aldopentosen, Aldofuranosen, Aldopyranosen, Glycosaminen der Aldopyranosen, dimeren reduzierenden Aldopyranosen und den daraus gebildeten Glycosaminen, dimeren reduzierenden Aldofuranosen und den daraus gebildeten Glycosaminen, trimeren Kohlenhydraten, Glucuronsäuren, Aldonsäuren oder den daraus gebildeten Lactonen. Insbesondere eignen sich im Rahmen der Erfindung Kohlenhydrate, die ausgewählt sind aus der Gruppe bestehend aus Allose, Altrose, Galactose, Glucose, Gulose, Idose, Mannose, Talose, Glucosamin, Galactosamin, Mannosamin, Maltose, Cellobiose, Gentiobiose, Lactose, Lactosamin, Melezitose, Panose, Erythrose, Threose, Ribose, Arabinose, Xylose, Lyxose, Glucuronsäure, Gluconsäure, Glucono-1,5-lacton, 1',2-O-lsopropyliden-D-isosaccarinlacton, Xylonsäurer-γ-lacton, Ribonsäurer-γ-lacton, Gulonsäure-γ-lacton, Mannonsäure-γ-lacton, Erythronsäure-γ-lacton, Glucoheptonsäure-γ-lacton und Glucooctansäure-γ-lacton.

Die Derivatisierung der Pyranosen kann an den Positionen 1 (anomeres Zentrum), 2 und/oder 6 erfolgen, die der Furanosen an den Positionen 1 (anomeres Zentrum) 2 oder 5.

Erfindungsgemäß können einzelne Anomere sowie Anomerengemische Einsatz finden.

In einer weiteren erfindungsgemäßen Ausführungsform ist wenigstens eine der reaktiven Komponenten eine Aminosäure oder von einer solchen abgeleitet. Sowohl die Amino-Gruppe als auch die Carboxyl-Gruppe der Aminosäure sind einer leichten Derivatisierung zugängig. So kann letztere beispielsweise mit komplexen Metallhydriden, wie Lithiumaluminiumhydrid, Lithiumalkoxyaluminiumhydriden oder Diisobutylaluminiumhydrid bei niedrigen Temperaturen zu den entsprechenden Aldehyden reduziert werden. Aufgrund der bereits im Molekül enthaltenen Carboxyl- und Amino-Gruppen ist jedoch in vielen Fällen eine weitere Derivatisierung nicht notwendig.

Aminosäuren, die als erfindungsgemäße Komponenten Einsatz finden können, sind ausgewählt aus der Gruppe bestehend aus Glycin, Alanin, Valin, Leucin, Serin, Threonin, Cystin, Cystein, Methionin, Arginin, Lysin, Asparagin, Asparaginsäure, Glutamin, Glutaminsäure, Phenylalanin, Tyrosin, Prolin, Tryptophan und Histidin.

Neben den Aminosäuren können auch Di-, Tri-, Tetra-, Oligo- und Polypeptide eingesetzt werden, soweit diese die erfindungsgemäßen Anforderungen an das Molekulargewicht erfüllen.

In einer weiteren erfindungsgemäßen Ausführungsform ist wenigstens eine der reaktiven Komponenten ein Vitamin oder von einem solchen abgeleitet.

Der Begriff Vitamin bezeichnet im allgemeinen keine konkrete chemische Stoffklasse, sondern vielmehr alle organischen Wirkstoffe, die für den normalen Stoffwechsel im menschlichen und tierischen Organismus zwar unentbehrlich sind, von diesem jedoch nicht synthetisiert werden können. Daher ist der Begriff Vitamin im Rahmen der vorliegenden Erfindung auf Verbindungen und deren Derivate beschränkt, die ausgewählt sind aus der Gruppe bestehend aus Retinol, Riboflavin, Tocopherol, Pantothensäure, Biotin, Folsäure, Linolsäure, Arachidonsäure, Pyridoxamin, Nicotinsäure und Ascorbinsäure. Diese Verbindungen verfügen teilweise über die geforderten Funktionalitäten, um als reaktive Komponente (A) oder (B) eingesetzt zu werden, so dass eine weitere Derivatisierung oftmals nicht notwendig ist. In einer weiteren erfindungsgemäßen Ausführungsform ist wenigstens eine der Komponenten ein UV-Filter oder von einem solchen abgeleitet.

Solche Verbindungen schützen das Haar vor ultravioletter Strahlung und bewahren es vor einer damit verbundenen Schädigung. Die als UV-Filter bezeichneten Wirkstoffe verfügen teilweise über die geforderten Funktionalitäten, so dass eine weitere Derivatisierung oftmals nicht notwendig ist. In vielen Fällen kann durch die Derivatisierung jedoch eine geeignete Funktionalisierung der Wirkstoffe erzielt werden. Erfindungsgemäß anwendbare UV-Filter sind beispielsweise ausgewählt aus der Gruppe bestehend aus Derivaten des Cyanurchlorids, 2-Hydroxy-4-methoxy-benzophenon, 4-Aminobenzoesäure, 3,3,5-Trimethylcyclohexylsalicylat, 3-lmidazol-4-yl-acrylsäure, 4-Aminobenzoesäure-1-glycerylester, Salicylsäure-2-ethyl-hexylester, 4-Hydroxy-4-methoxy-4-methylbenzophenon.

Die zur Derivatisierung der Wirkstoffe zu treffenden Maßnahmen sind im Hinblick auf die einzelnen Wirkstoffe individuell zu wählen. Generell sind diese Maßnahmen im Stand der Technik vorbeschrieben und dem Fachmann bekannt.

Insbesondere eignen sich zur Amino-Derivatisierung der Wirkstoffe Alkylen- und Arylendiamine gemäß der Formel (I)

H₂N-R-NH₂ (I)

R repräsentiert darin
- eine 2 bis 12, bevorzugt 1 bis 8, besonders bevorzugt 1 bis 5 Kohlenstoffe enthaltende gesättigte oder ungesättigte, verzweigte oder lineare, aliphatische oder alicyclische Alkylen-Gruppe,
- eine 5 bis 12, bevorzugt 5 bis 10, besonders bevorzugt 5 bis 6 Kohlenstoffe enthaltende Arylen-Gruppe
- eine Heteroarylen-Gruppe mit 3 bis 5 Kohlenstoffatomen und bis zu 2 Stickstoffatomen,
- ein Strukturelement gemäß der nachfolgenden Formel II, 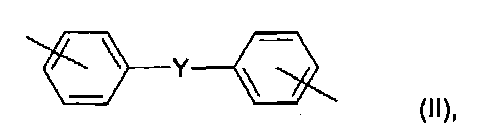
Y repräsentiert darin
- eine aliphatische 1 bis 5 Kohlenstoffe, vorzugsweise 1 bis 2 Kohlenstoifie enthaltende Alkylen-Gruppe,
- eine 2 bis 5 Kohlenstoffe enthaltende Alkenylen-Gruppe,
- eine Sulfid-Gruppe (-S-) oder Disulfid-Gruppe (-S-S-).

Besonders bevorzugt sind Amine, die ausgewählt sind aus der Gruppe, bestehend aus 1.2-Diaminoethan, 1,2- oder 1,3-Diaminopropan, 1,2-, 1,3- oder 1,4-Diaminobutan, 1,5-Diaminopentan, 2,2-Dimethyl-1,3-diaminopropan, Hexamethylendiamin, 1,7-Diamino-heptan, 1,8-Diaminooctan, Trimethyl-1,6-diaminohexan, 1,9-Diaminononan, 1,10-Diaminodecan, 1,12-Diaminododecan, 1,2-Diaminocyclohexan, 1,4-Diaminocyclohexan, 1,3-Cyclohexanbis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diaminopyridin, 2,3-Diaminopyridin, 3,4-Diaminopyridin, 2,4-Diaminopyrimidin, 4,5-Diaminopyrimidin und 4,6-Diaminopyrimidin.

Vorausgesetzt eine Komponente leitet sich von einem Wirkstoff ab, so kann sich auch deren komplementäre Komponente ebenfalls von einem Wirkstoffmolekül ableiten. Letzteres führt zu einer Fixierung zweier identischer oder verschiedener Wirkstoffeinheiten am Haar.

Alternativ kann eine von einem Wirkstoff abgeleitete reaktive Komponente jedoch auch mit einer weiteren reaktiven Komponente reagieren, die sich im Gegensatz zur ersten Komponente nicht von einem Wirkstoff ableitet. In diesem Fall wird durch die Reaktion in/an/auf dem Haar nur die Wirkstoffeinheit der ersten Komponente aufgebracht, während die andere Komponente ausschließlich der Immobilisierung der ersten Komponente dient.

In einer besonderen Ausgestaltung der vorliegenden Erfindung kann eine erste Komponente, die sich vorzugsweise von keinem Wirkstoff ableitet, mehrere gleiche oder verschiedene Funktionalitäten aufweisen, die eine Knüpfung der genannten Bindungstypen mit mehreren reaktiven Komponenten ermöglichen.

Der ersten Komponente kommt dabei die Rolle eines Linkers oder Vernetzers zu. Zur besseren Erläuterung dieser Ausgestaltung soll die Reaktion gemäß Gleichung III dienen, die jedoch nur eine von zahlreichen Varianten darstellt und auf welche der Gegenstand der Erfindung nicht zu beschränken ist.

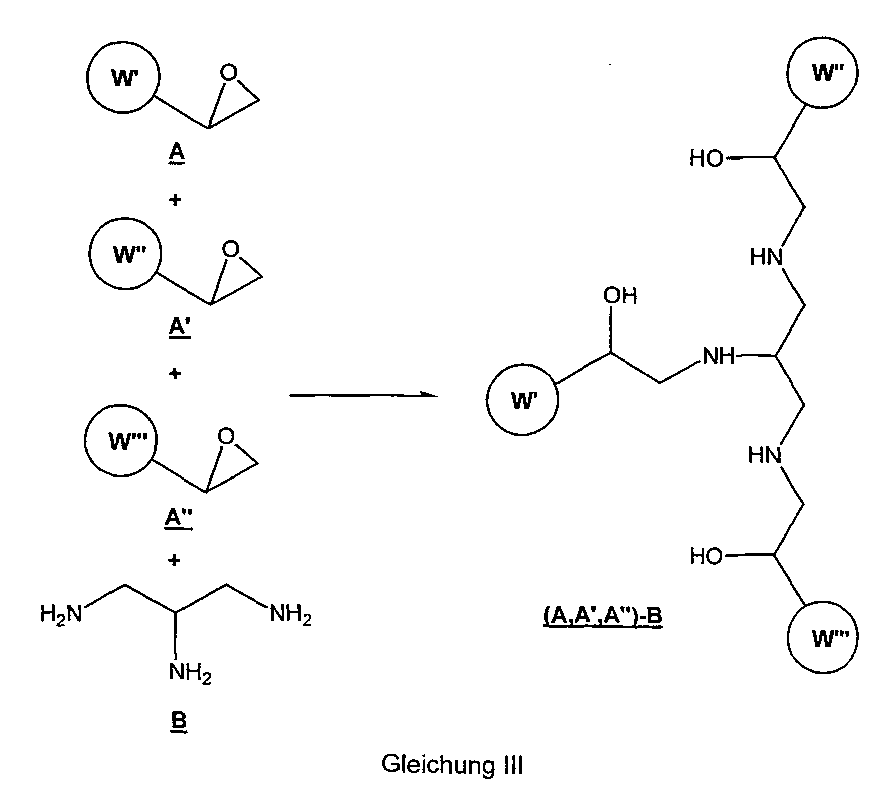

Als vorzugsweise nicht von einem Wirkstoff abgeleitete reaktive Komponente (B) eignen sich Verbindungen, die ausgewählt sind aus der Gruppe bestehend aus:
(I) Aldehyden mit bis zu 25, bevorzugt 2 bis 20, besonders bevorzugt 2 bis 13 Kohlenstoffatomen.
   Bevorzugt sind Aldehyde der Formel R¹[-CHO]_{n'}, wobei n' = 1, 2 oder 3, vorzugsweise 1 oder 2 ist und R¹ ausgewählt ist aus der Gruppe bestehend aus
   - linearen oder verzweigten, gesättigten oder ungesättigten, aliphatischen oder alicyclischen Alkyl-Gruppen mit 1 bis 12, vorzugsweise 2 bis 8, besonders bevorzugt 2 bis 4 Kohlenstoffatomen;
   - Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gruppen; und
   - Heteroaryl-Gruppen mit 3 bis 8 Kohlenstoffatomen und einem oder zwei Heteroatom(en) ausgewählt aus N, O und S, die substituiert sein können mit C₁-C₆-Alkyl-und/oder C₂-C₆-Alkoxy-Gruppen,
   wobei die Alkyl-, Aryl- und Heteroaryl-Gruppen gegebenenfalls mit wenigstens einer Oxo-, Formyl-, C₁-C₈-Acyl- oder C₆-C₁₀-Arylcarbonyl-Gruppe substituiert sein können.
   Besonders bevorzugt sind Aldehyde, die ausgewählt sind aus der Gruppe bestehend aus Benzaldehyd, Zimtaldehyd, *trans*-Stilbencarboxaldehyd, Helicin, Dihydroxyformylphenylglycosid, Diformylpiperazin, Pyridincarboxaldehyd, Pyrrolcarboxaldehyd, Pyridoxal, Phthaldialdehyd, Citronellal und Valeraldehyd.
(II) Ketonen mit bis zu 40, bevorzugt 3 bis 26, besonders bevorzugt 3 bis 14 Kohlenstoffatomen.
   Bevorzugt sind Ketone der Formel R²-(C=O)-R³, wobei R² und R³ unabhängig voneinander ausgewählt sind aus der Gruppe bestehend aus
   - linearen oder verzweigten, gesättigten oder ungesättigten, aliphatischen oder alicyclischen Alkyl-Gruppen mit 1 bis 12, vorzugsweise 2 bis 8, besonders bevorzugt 2 bis 4 Kohlenstoffatomen;
   - Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gruppen; und
   - Heteroaryl-Gruppen mit 3 bis 8 Kohlenstoffatomen und einem oder zwei Heteroatom(en) ausgewählt aus N, O und S, die gegebenenfalls substituiert sein können mit C₁-C₆-Alkyl- und/oder C₂-C₆-Alkoxy-Gruppen,
   wobei die Alkyl-, Aryl- und Heteroaryl-Gruppen gegebenenfalls mit wenigstens einer Oxo-, Formyl-, C₁-C₈-Acyl- oder C₆-C₁₀-Arylcarbonyl-Gruppe substituiert und unabhängig davon R² und R³ über eine Einfachbindung, Kohlenstoff-, Stickstoff-, Schwefel- und/oder Sauerstoffatome, unter Bildung eines 4 bis 8 Atome enthaltenden Rings untereinander verbunden sein können.
   Besonders bevorzugt sind die Ketone, die ausgewählt sind aus der Gruppe bestehend aus Pentanon, Hexanon, Heptanon, Octanon, 3-Penten-2-on, 5-Hexen-2-on, 4-Hexen-3-on, 5-Methyl-3-hexen-2-on, 5-Methyl-5-hexen-2-on, 6-Methyl-5-hepten-2-on, 3-Nonen-2-on, Phoron, Nerylaceton, Geranylaceton, Adamantylketon, Campher, Cyclohexenon, Benzochinon, Benzylaceton, Acetophenon, Valerophenon, 1,2-Diacetylbenzol, 4,4'-Diacetylbiphenyl und 1'-Acetonaphthenon.
(III) Aminen, mit bis zu 25, bevorzugt 2 bis 20, besonders bevorzugt 2 bis 13 Kohlenstoffatomen,
   Bevorzugt sind Amine der Formel R⁴[-NH-R⁵]_{n"}
   wobei n" = 1,2 oder 3, vorzugsweise 1 oder 2 ist
   und R⁴ ausgewählt ist aus der Gruppe bestehend aus
   - linearen oder verzweigten, gesättigten oder ungesättigten, aliphatischen oder alicyclischen Alkyl-Gruppen mit 1 bis 12, vorzugsweise 2 bis 8, besonders bevorzugt 2 bis 4 Kohlenstoffatomen;
   - Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gi-uppen; und
   - Heteroaryl-Gruppen mit 3 bis 8 Kohlenstoffatomen und einem oder zwei Heteroatom(en) ausgewählt aus N, O und S, die substituiert sein können mit C₁-C₆-Alkyl- und/oder C₂-C₆-Alkoxy-Gruppen,
   wobei die Alkyl-, Aryl- und Heteroaryl-Gruppen gegebenenfalls mit wenigstens einer Oxo-, Formyl-, C₁-C₆-Acyl- oder C₆-C₁₀-Arylcarbonyl-Gruppe substituiert sein können,
   während R⁵ ausgewählt ist aus Wasserstoff.

Besonders bevorzugt sind Amine, die ausgewählt sind aus der Gruppe bestehend aus Butylamin, Amylamin, Hexylamin, Heptylamin, Octylamin, Nonylamin, Decylamin, Undecylamin, Dodecylamin, Tridecylamin, 1-Tetradecylamin, Isobutylamin, *tert*-Butylamin, 1-Methylbutylamin, 1-Ethylpropylamin, 2-Methylbutylamin, Isoamylamin, 1,2-Dimethylpropylamin, *tert*-Amylamin, 1,3-Dimethylbutylamin, 3,3-Dimethylbutylamin, 2-Aminoheptan, 3-Aminoheptan, Cyclopentylamin, Cyclohexylamin, Cyclohexanmethylamin, 3-Methoxypropylamin, 3-Ethoxypropylamin, 3-Butoxypropylamin, Tetrahydrofurfurylamin, Ethanolamin, 3-Amino-1-propanol, DL-2-Amino-1-propanol, DL-1-Amino-2-propanol, 4-Amino-1-butanol, 2-Amino-1-butanol, 2-Amino-2-methyl-1-propanol, 5-Amino-1-propanol, DL-2-Amino-1-pentanol, 6-Amino-1-hexanol, DL-2-Amino-1-hexanol, Aminocyclohexanol, Serinol, 1-Amino-1-deoxy-D-sorbitol, D-Gallosamin, D-Glucosamin, D-Mannosamin, Benzylamin, Phenylethylamin, 3-Phenyl-1-propylamin, 3,3-Diphenylpropylamin, 4-Phenylbutylamin, N-Phenylethylendiamin, 2-Amino-1-phenylethanol, 2-Methoxybenzylamin, 2-Methoxyphenethylamin und 2-Ethoxybenzylamin.

Für den Fall, dass die erfindungsgemäßen Mehrkomponentensysteme zur Behandlung lebendiger Haare verwendet werden, ist die Auswahl der geeigneten Komponente auf solche beschränkt, die Körper und Gesundheit des Anwenders nicht negativ beeinträchtigen. Bei der Behandlung von separierten Haaren, wie z.B. im Fall von Echthaarperücken, sind die Anforderungen an die toxikologischen Eigenschaften der Verbindungen deutlich geringer.

Beispielsweise können Verbindungen gemäß den nachfolgenden Formeln III bis VII oder deren Hydrohalogenide, bevorzugt deren Hydrochloride und/oder Verbindungen gemäß der nachfolgenden Formeln VIII verwendet werden,

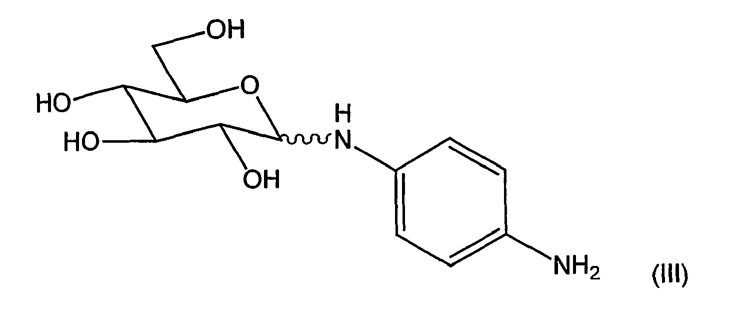

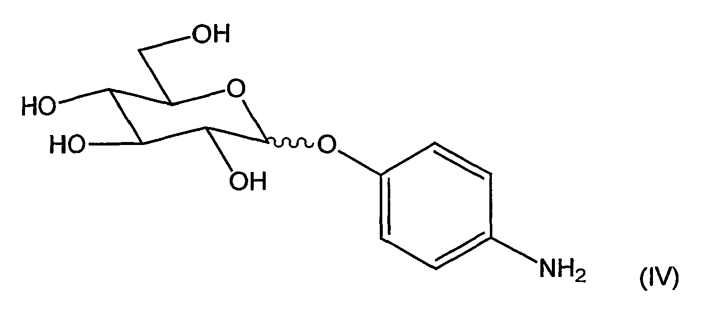

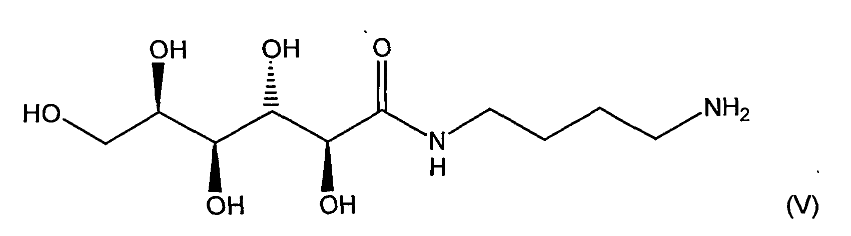

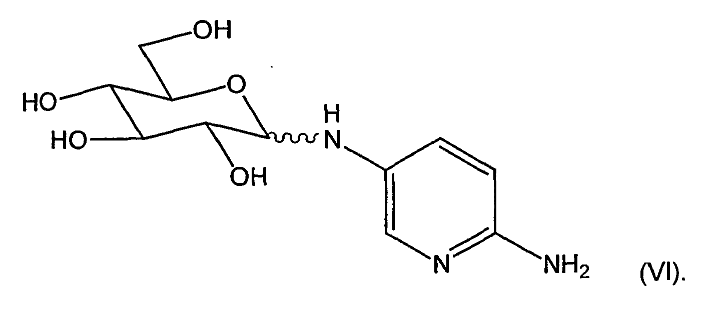

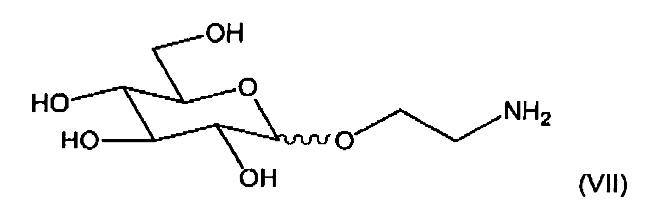

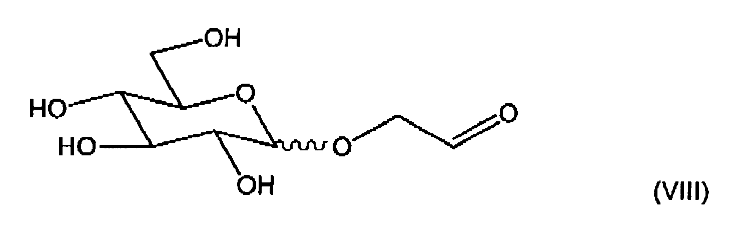

In einer weiteren Ausführungsform betrifft die Erfindung eine Zusammensetzung, umfassend die oben angeführten Komponenten zur Behandlung von Fasern, insbesondere von menschlichen und tierischen Haaren Durch die Behandlung kann eine dauerhafte und positive Beeinflussung der Eigenschaften wie Nass- und Trockenkämmbarkeit, Halt, Feuchtigkeit, Festigkeit, Fülle, Glanz, Taktilität und elektrostatischer Eigenschaften sowie erhöhte Resistenz gegen UV- und IR-Strahlung und Wärmeeinwirkung erzielt werden. Insbesondere ist die Erfindung zur positiven Beeinflussung des Haarvolumens geeignet.

Die Zusammensetzung kann zusätzlich zu den reaktiven Komponenten einen Träger enthalten. Die Komponenten liegen darin in einer Gesamtmenge von 0,2 bis 20 Gew.%, bevorzugt 1 bis 20 Gew.-%, besonders bevorzugt 3 bis 10 Gew.-%, bezogen auf die Gesamtzusammensetzung vor.

Geeignete Träger sind fest, flüssig, gelförmig oder pastös und bestehen bevorzugt aus wässrigen Systemen, natürlichen oder synthetischen Ölen, Wasser-in-Öl oder Öl-in-Wasser-Emulsivnen. Derartige Systeme sind dem Haarkosmetik-Fachmann bekannt. Der Träger kann zudem übliche Zusatzstoffe wie Verdicker, Tenside, Rheologiehilfsmittel, Antioxidantien, Stabilisatoren, Farb-, Aroma-, Geruchs-, Effektstoffe und dergleichen enthalten.

Die erfindungsgemäßen Mehrkomponenten-Systeme können als Bestandteil von Haarbehandlungsmitteln wie Shampoos, Conditionern, Spülungen, Aerosolen und Gelen Anwendung finden. Üblicherweise umfassen solche Mittel die Komponenten (A) und (B) in einer Konzentration von 0,001 bis 10 Gew.-%, bevorzugt 0,1 bis 8 Gew.-%, besonders bevorzugt 0,5 bis 5 Gew.-% der Komponente (A) und 0,001 bis 10 Gew.-%, bevorzugt 0,1 bis 8 Gew.-%, besonders bevorzugt 0,5 bis 5 Gew.-%, der Komponente (B), bezogen auf die Gesamtzusammensetzung des Mittels.

Die Applikation der Komponenten kann so erfolgen, dass diese unabhängig voneinander und nacheinander auf das zu behandelnde Haar aufgetragen werden. Hierzu wird zunächst die eine Komponente aufgebracht, eine bestimmte Zeit einwirken gelassen, anschließend wird das Haar mit der/den komplementären Komponente(n) behandelt. Alternativ kann ein Vermischen der Komponenten kurz vor der Anwendung erfolgen. Die Mischung wird dann zügig auf das Haar aufgebracht. Diese Applikationsform setzt jedoch voraus, dass die beiden Komponenten nicht unmittelbar nach dem Vermischen vollständig miteinander reagieren. Die Bildung der resultierenden Wirkkomponente (A-B) erfolgt in beiden Fällen an dem behandelten Haar.

In einer Darreichungsform werden die Komponenten und gegebenenfalls weitere Bestandteile der zur Anwendung kommenden Zusammensetzung getrennt voneinander in einem Kit-of-parts zur Verfügung gestellt. Die einzelnen Komponenten können in einem geeigneten Träger gemischt, gelöst, dispergiert oder emulgiert vorliegen. Nach dem Zusammenfügen der Teile des Kit-of-parts erhält man die zuvor beschriebene Zusammensetzung.

Die Erfindung wird durch die folgenden Beispiele näher erläutert, ohne sie jedoch darauf zu beschränken.

### Beispiele

Zur Verdeutlichung des erfindungsgemäßen Effektes wurden mehrere reaktive Komponenten, gemäß der Erfindung synthetisiert und zunächst auf ihre Reaktivität getestet.

Darstellung von geeigneten reaktiven Komponenten:

### 1,4-Diaminophenyl-β-D-glucopyranose (III)

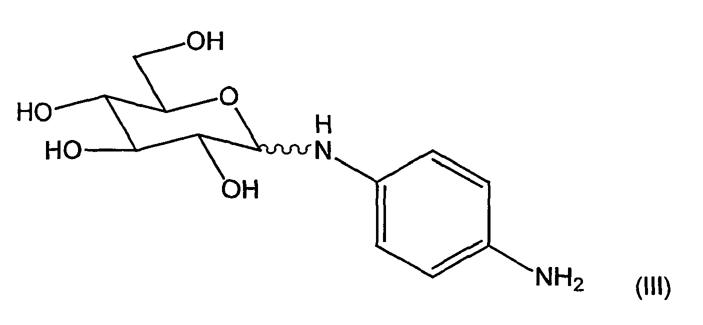

Die Darstellung der Verbindung III erfolgte gemäß den Vorschriften von: Matsubara S., Boyel W.C., J. Immunol. 96(1), 1966, S. 25-28.

### 2,5-Diaminopyridin-β-D-glucopyranose (VI)

1,1 g 2,5-Diaminopyridin werden in 10 mL Ethanol gelöst und zu einer Suspension von 1,80 g wasserfreier Glucose in 10 mL Ethanol zugegeben. Das Reaktionsgemisch wird 4 h unter Rückfluss gekocht und nach Reaktionsende auf Raumtemperatur abgekühlt, wobei das Produkt kristallisiert. Durch Absaugen des Feststoffs und waschen mit eiskaltem Methanol konnte die Verbindung gemäß Formel VI erhalten werden (DC: Kieselgel 60 F₂₅₄ auf Plastikfolie, Merck, R_{f} = 0,65, Isopropanol/H₂O = 9/1).

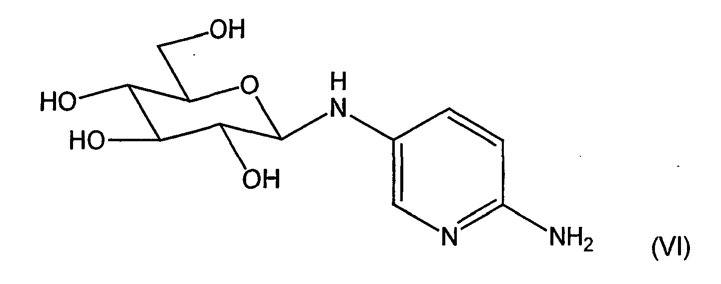

### 4-Aminobutylgluconsäureamid (V)

0,97 g 1,4-Diaminobutan werden in 30 mL Methanol aufgenommen, portionsweise werden 1,78 g D(+)-Gluconsäure-8-lacton zugegeben und bei Raumtemperatur gerührt. Nach etwa 10 min Reaktionszeit erfolgt die Kristallisation der Verbindung gemäß Formel V. Nach dem Absaugen der Kristalle wird zur weiteren Reinigung mit Methanol gewaschen (DC: Kieselgel 60 F₂₅₄ auf Plastikfolie, Merck, R_{f} = 0,12, MeOH/H₂O/NEt₃ = 70/29/1).

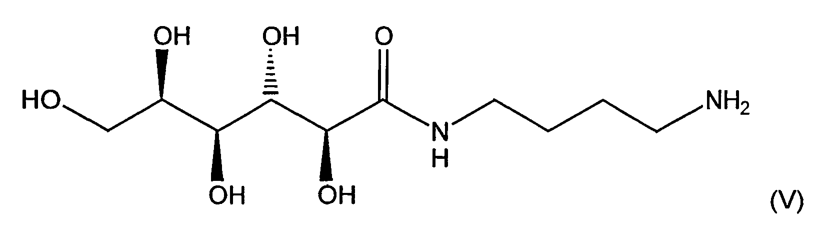

### 4-Aminophenyl-β-D-glucopyranose (IV)

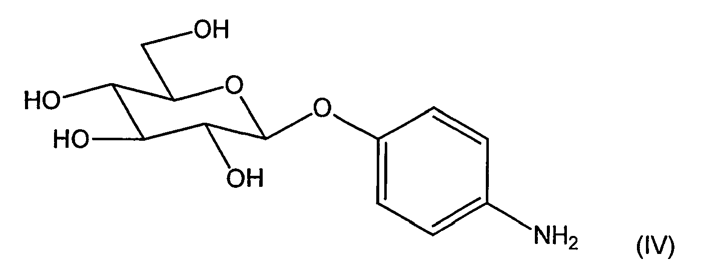

Die Verbindung IV wird durch die Fluka AG kommerziell vertrieben.

### 2-Aminoethyl-α,β-D-glucopyranose (VII)

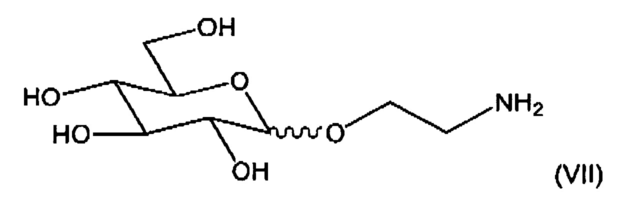

Die Darstellung der Verbindung VII erfolgte gemäß den Vorschriften von ;
1. B. Pucci, B. Ragonnet, AA. Pavia, Eur. Polym. J., 1988, Vol. 24, No. 11, 1087-1091.
2. S.M. Dimick, S.C. Powell, S.A. Mc Mahon, D.N. Moothoo, J.H. Naismuth, E.J. Toone, J. Am. Chem. Soc., 1999, 121, 10286-10296.

### Formylmethylglycosid (VIII)

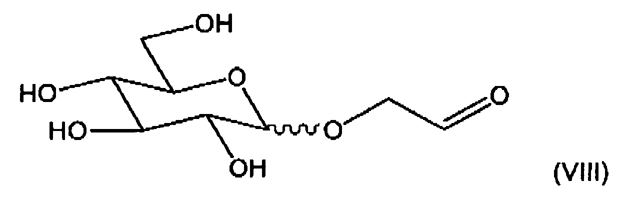

Die Darstellung von Formylmethylglycosid (VIII) erfolgte gemäß einer Vorschrift von K.R. Holme, L.D. Hall, Carbonhydr. Res., 1992, 225(2), 291-306 und Tetrahedr. Lett., 1966, 36, 4273-4278.

### Untersuchungen der Reaktivität einzelner Zweikomponentensysteme

Die Durchführung der Reaktionen erfolgte fernab des Haars und dient lediglich einem Nachweis der Reaktivität der erfindungsgemäßen Mehrkomponentensysteme.

### Untersuchung der Reaktivität von 4-Aminobutylgluconsäureamid (V) als Komponente (A) mit Citronellal als Komponenten (B):

1,21 g der Verbindung gemäß Formel V werden in 20 mL DMF gelöst und mit einer katalytischen Menge Triethylamin versetzt, Nach Zutropfen von 0,84 g Citronellal in 10 mL DMF wird das Reaktionsgemisch für 30 min bei 30 °C gerührt, anschließend wird das Lösungsmittel im Vakuum entfernt und das rosafarbene Rohprodukt mit kaltem Ether gewaschen und im Hochvakuum getrocknet (DC: Kieselgel 60 F₂₅₄ auf Plastikfolie, Merck, R_{f} = 0,22, Aceton/H₂O = 98/2).

### Untersuchung der Reaktivität von 2,5-Diaminopyridin-β-D-glucopyranose (VI) als Komponente (A) mit Citronellal als Komponente (B):

1,36 g der Verbindung gemäß Formel VI werden in 20 mL DMF gelöst. Nach dem Zutropfen von 0,84 g Citronellal in 10 mL DMF wird das Reaktionsgemisch für 30 min bei 30 °C gerührt, anschließend das Lösungsmittel im Vakuum entfernt und das rosafarbene Rohprodukt mit kaltem Ether gewaschen und im Hochvakuum getrocknet (DC: Kieselgel 60 F₂₅₄ auf Plastikfolie, Merck, R_{f} = 0,25, Aceton/H₂O = 95/5).

**Anwendungsbeispiel 1**

| Inhaltsstoff | Menge [Gew.-%] |
|---|---|
| *Zusammensetzung umfassend Komponente A* | |
| **4-Aminobutylgluconsäureamid (V)** | 2,0 |
| Cetearylalkohol | 5,5 |
| Ceteareth-20 | 2,0 |
| Sodium Laureth Sulfat | 1,5 |
| Polyquaternium-10 | 0,8 |
| Cocoamidopropylbetain | 0,5 |
| Ammoniumhydroxid | q.s. |
| Aqua | ad 100,0 |
| Die Formulierung wird durch Zugabe einer ausreichenden Menge an Ammoniak auf einen pH-Wert von 9,0 eingestellt. | |
| *Zusammensetzung umfassend Komponente B* | |
| **Citronellal** | 1,5 |
| Acrylates Copolymer | 0,5 |
| Sodium Laureth Sulfat | 0,3 |
| Puffersubstanzen (Zitronensäure) | q.s. |
| Aqua | ad 100,0 |

**Anwendungsbeispiel 2**

| Inhaltsstoff | Menge [Gew.-%] |
|---|---|
| *Zusammensetzung umfassend Komponente A* | |
| **Formylmethylglucosid (VIII)** | 1,5 |
| Acrylates Copolymer | 0,5 |
| Natrium Laureth Sulfat | 0,3 |
| Puffersubstanzen (Zitronensäure) | q.s. |
| Aqua | ad 100,0 |
| *Zusammensetzung umfassend Komponente B* | |
| **Aminobutylgluconsäureamid (V)** | 2,0 |
| Cetearylalkohol | 5,5 |
| Ceteareth-20 | 2,0 |
| Natrium Laureth Sulfat | 1,5 |
| Polyquaternium-10 | 0,8 |
| Cocoamidopropylbetaine | 0,5 |
| Aqua | ad 100,0 |

## Patentansprüche

1. Verfahren zur Erzielung einer dauerhaften und positiven Beeinflussung des Volumens, der Nass- und Trockenkämmbarkeit, des Haltes, der Feuchtigkeit, der Fülle, des Glanzes, der Festigkeit, der Taktilität, der elektrostatischen Eigenschaften, der erhöhten Resistenz gegen UV- und IR-Strahlung und Wärmeeinwirkung von Fasern, insbesondere von menschlichen und tierischen Haaren, durch Immobilisierung von Wirkstoffen an Fasern, durch deren Behandlung mit einem Mehrkomponenten-System, umfassend
- wenigstens eine reaktive Komponente (A) mit einem Molekulargewicht ≤1000 Dalton, die wenigstens eine funktionelle Gruppe aufweist, die ausgewählt ist aus primären Amino-Gruppen,
- wenigstens eine komplementäre reaktive Komponente (B) mit einem Molekulargewicht ≤1000 Dalton, die wenigstens eine funktionelle Gruppe aufweist, die ausgewählt ist aus Carbonyl-Gruppen,
wobei die Komponenten (A) und (B) miteinander unter Bildung einer Schiffschen Base reagieren, und wobei sich wenigstens eine der Komponenten (A) und (B) von einem Wirkstoff ableitet, der ausgewählt ist aus der Gruppe, bestehend aus Kohlenhydraten, Aminosäuren, Vitaminen und UV-Filtern, wobei die in den reaktiven Komponenten enthaltenen funktionellen Gruppen durch geeignete Derivatisierung in die Wirkstoffmolekole eingeführt werden können.

2. Verfahren nach Anspruch 1, **dadurch gekennzeichnet, dass** das Kohlenhydrat, von dem die reaktive Komponente (A) und/oder (B) abgeleitet ist, ausgewählt ist aus der Gruppe bestehend aus Aldohexosen, Aldopentosen, Aldofuranosen, Aldopyranosen, Glycosaminen der Aldofuranosen, dimeren reduzierenden Aldopyranosen und den daraus gebildeten Glycosaminen, dimeren reduzierenden Aldofuranosen und den daraus gebildeten Glycosaminen, trimeren Kohlenhydraten, Glycuronsäuren, Aldonsäuren oder den sich daraus ableitenden Lactonen.

3. Verfahren nach Anspruch 2, **dadurch gekennzeichnet, dass** das Kohlenhydrat von dem die reaktive Komponente (A) und/oder (B) abgeleitet ist, ausgewählt ist aus der Gruppe bestehend aus Allose, Altrose, Galactose, Glucose, Gulose, Idose, Mannose, Talose, Glucosamin, Galactosamin, Mannosamin, Maltose, Cellobiose, Gentiobiose, Lactose, Lactosamin, Melezitose, Panose, Erythrose, Threose, Ribose, Arabinose, Xylose, Lyxose, Glucuronsäure, Gluono-1,5-lacton, 1',2-O-Isopropyliden-D-isosaccarinlacton, Xylonsäure-γ-lacton, Ribonsäure-γ-lacton, Gulonsäure-γ-lacton, Mannonsäure-γ-lacton, Erythronsäure-γ-lacton, Glucoheptansäure-γ-lacton, Glucooctansäure-γ-lacton.

4. Verfahren nach irgendeinem der Ansprüche 2 und 3, **dadurch gekennzeichnet, dass** die Pyranosen an der Position 1 (anomeres Zentrum), 2 und/oder 6, die Furanosen an der Positionen 1 (anomeres Zentrum), 2 oder 5 derivatisiert sind.

5. Verfahren nach irgendeinem der vorherigen Ansprüche, **dadurch gekennzeichnet, dass** zur Amino-Derivatisierung der Wirkstoffe Amine gemäß der nachfolgenden Formel
H₂N-R-NH₂ (I)
verwendet werden,
wobei R
- eine 2 bis 12 Kohlenstoffatome enthaltende, gesättigte oder ungesättigte, verzweigte oder lineare, aliphatische oder alicyclische Alkylen-Gruppe,
- eine 5 bis 10 Kohlenstoffe enthaltenden Arylen-Gruppe,
- eine 3 bis 5 Kohlenstoffatome enthaltende Heteroarylen-Gruppe mit bis zu 2 Stickstoffatomen,
- ein Strukturelement gemäß der nachfolgenden Formel (II) ist, 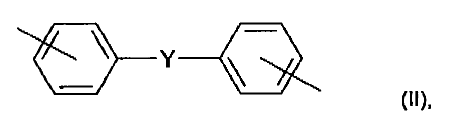 worin Y ausgewählt ist aus
- einer aliphatische 1 bis 5 Kohlenstoffe enthaltende Alkylen-Gruppe,
- einer 2 bis 5 Kohlenstoffe enthaltende Alkenylen-Gruppe,
- einer Sulfid-Gruppe (-S-) oder einer Disulfd-Gruppe (-S-S-).

6. Verfahren nach Anspruch 5, **dadurch gekennzeichnet, dass** die zur Amino-Derivatisierung der Wirkstoffe verwendeten Amine ausgewählt sind aus der Gruppe bestehend aus 1,2-Diaminoethan, 1,2- oder 1,3-Diaminopropan, 1,2-, 1,3- oder 1,4-Diaminobutan, 1,5-Diaminopentan, 2,2-Dlmethyl-1,3-diaminopropan, Hexamethylendiamin, 1,7-Diamino-heptan, 1,8-Diaminooctan, Trimethyl-1,6-diaminohexan, 1,9-Diaminononan, 1,10-Diaminodecan, 1,12-Diaminododecan, 1,2-Diaminocyclohexan, 1,4-Diaminocyclohexan, 1,3-Cyclohexanbis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diaminopyridin, 2,3-Diaminopyridin, 3,4-Diaminopyridin, 2,4-Diaminopyrimidin, 4,5-Diaminopyrimidin und 4,6-Diaminopyrimidin.

7. Verfahren nach Anspruch 1, **dadurch gekennzeichnet, dass** die reaktive Komponente (A) ein Amino-derivatisiertes Kohlenhydrat gemäß der nachfolgenden Formel III oder ein daraus abgeleitetes Hydrohalogenid ist

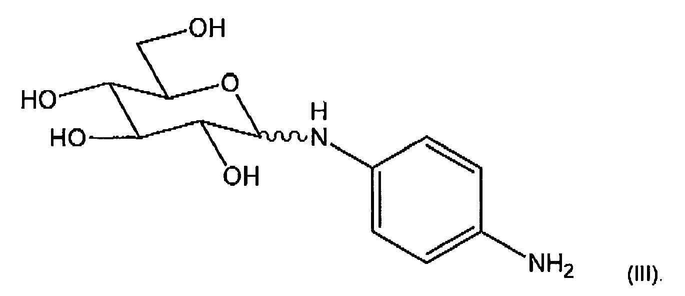

8. Verfahren nach Anspruch 1, **dadurch gekennzeichnet, dass** die reaktive Komponente (A) ein Amino-derivatisiertes Kohlenhydrat gemäß der nachfolgenden Formel IV oder ein daraus abgeleitetes Hydrohalogenid ist

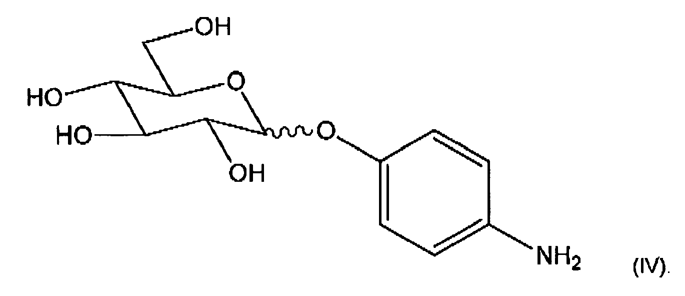

9. Verfahren nach Anspruch 1, **dadurch gekennzeichnet, dass** die reaktive Komponente (A) ein Amino-derivatisieries Kohlenhydrat gemäß der nachfolgenden Formel V oder ein daraus abgeleitetes Hydrohalogenid ist

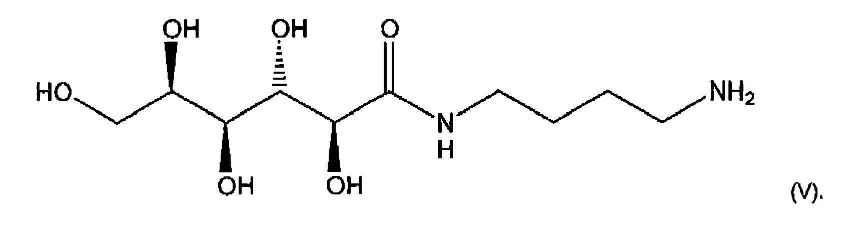

10. Verfahren nach Anspruch 1, **dadurch gekennzeichnet, dass** die reaktive Komponente (A) ein Amino-derivatisiertes Kohlenhydrat gemäß der nachfolgenden Formel VI oder ein daraus abgeleitetes Hydrohalogenid ist

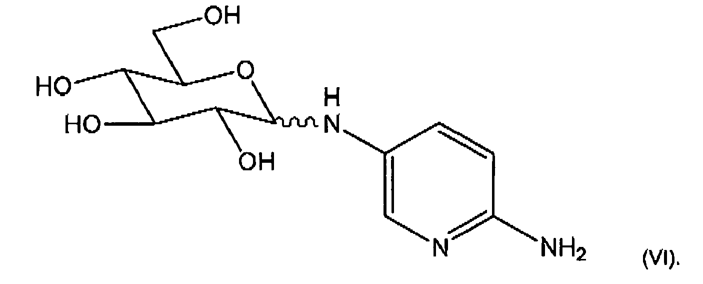

11. Verfahren nach Anspruch 1, **dadurch gekennzeichnet, dass** die reaktive Komponente (A) ein Amino-derivatisiertes Kohlenhydrat gemäß der nachfolgenden Formel VII oder ein daraus abgeleitetes Hydrohalogenid ist

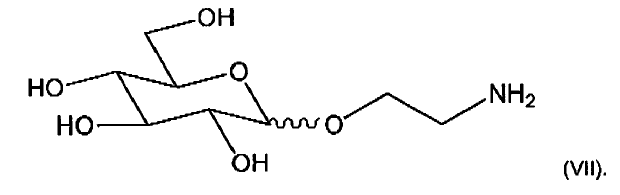

12. Verfahren nach Anspruch 1, **dadurch gekennzeichnet, dass** die reaktive Komponente (B) ein Carbonyl-derivatisiertes Kohlenhydrat gemäß der nachfolgenden Formel VIII ist

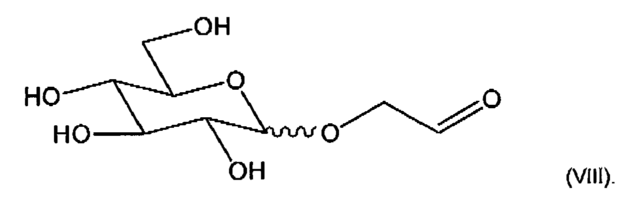

13. Verfahren nach Anspruch 1, **dadurch gekennzeichnet, dass** die Aminosäure, von der sich die reaktive Komponente (A) und/oder (B) ableitet, ausgewählt ist aus der Gruppe bestehend aus Glycin, Alanin, Valin, Leucin, Serin, Threonin, Cystin, Cystein, Methionin, Arginin, Lysin, Asparagin, Asparaginsäure, Glutamin, Glutaminsäure, Phenylalanin, Tyrosin, Prolin, Tryptophan und Histidin.

14. Verfahren nach Anspruch 1, **dadurch gekennzeichnet, dass** das Vitamin, von dem sich die reaktive Komponente (A) und/oder (B) ableitet, ausgewählt ist aus der Gruppe bestehend aus Retinol, Riboflavin, Tocopherol, Nicotinsäure und deren Derivate, Pantothensäure, Biotin, Folsäure, Linolsäure, Arachidonsäure, Ascorbinsäure, Pyridoxal und Pyridoxamin.

15. Verfahren nach Anspruch 1, **dadurch gekennzeichnet, dass** der UV-Filter, von dem sich die reaktive Komponenten (A) und/oder (B) ableitet, ausgewählt ist aus der Gruppe bestehend aus Derivaten des Cyanurchlorids, 2-Hydroxy-4-methoxy-benzophenon, 4-Aminobenzoesäure, 3,3,5-Trimethylcyclohexylsalicylat, 3-Imidazol-4-yl-acrylsäure, 4-Aminobenzoesäure-1-glycerylester, Salicylsäure-2-ethyl-hexylester und 4-Hydroxy-4-methoxy-4-methylbenzophenon.

16. Verfahren gemäß Anspruch 1, **dadurch gekennzeichnet, dass** die reaktive Komponente (A) ausgewählt ist aus primären Aminen und die reaktive Komponente (B) ausgewählt ist aus Aldehyden und Ketonen.

17. Verfahren nach Anspruch 16, **dadurch gekennzeichnet, dass** die Aldehyde bis zu 25 Kohlenstoffatome und die Ketone bis zu 40 Kohlenstoffatome enthalten können.

18. Verfahren nach irgendeinem der Anspruche 16 und 17, **dadurch gekennzeichnet, dass** die Aldehyde Verbindungen der Formel R¹[-CHO]ₙ sind, wobei n = 1, 2 oder 3 ist und R¹ ausgewählt ist aus der Gruppe bestehend aus
- linearen oder verzweigten, gesättigten oder ungesättigten, aliphatischen oder alicyclischen Alkyl-Gruppen mit 1 bis 12 Kohlenstoffatomen;
- Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gruppen; und
- Heteroaryl-Gruppen mit 3 bis 8 Kohlenstoffatomen und einem oder zwei Heteroatom(en) ausgewählt aus N, O und S, die substituiert sein können mit C₁-C₆-Alkyl- und/oder C₂-C₆-Alkoxy-Gruppen,
wobei die Alkyl-, Aryl- und die Heteroaryl-Gruppen gegebenenfalls mit wenigstens einer Oxo-, Formyl-, C₁-C₈-Acyl- oder C₆-C₁₀-Arylcarbonyl-Gruppe substituiert sein können.

19. Verfahren nach irgendeinem der Ansprüche 16 bis 18, **dadurch gekennzeichnet, dass** die Aldehyde ausgewählt sind aus der Gruppe bestehend aus Benzaldehyd, Zimtaldehyd, *trans*-Stilbencarboxaldehyd, Helicin, Dihydroxycarboxyphenylglycosid, Diformylpiperazin, Pyridincarboxaldehyd, Pyrrolcarboxaldehyd, Pyridoxal, Phthaldialdehyd, Citronellal und Valeraldehyd.

20. Verfahren nach irgendeinem der Ansprüche 16 und 17 **dadurch gekennzeichnet, dass** die Ketone Verbindungen der Formel R²-(C=O)-R³ sind, wobei R² und R³ unabhängig voneinander ausgewählt sind aus der Gruppe bestehend aus
- linearen oder verzweigten, gesättigten oder ungesättigten, aliphatischen oder alicyclischen Alkyl-Gruppen mit 1 bis 12 Kohlenstoffatomen;
- Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gruppen; und
- Heteroaryl-Gruppen mit 3 bis 8 Kohlenstoffatomen und einem oder zwei Heteroatom(en) ausgewählt aus N, O und S, die gegebenenfalls substituiert sein können mit C₁-C₆-Alkyl- und/oder C₂-C₆-Alkoxy-Gruppen,
wobei die Alkyl-, Aryl- und Heteroaryl-Gruppe gegebenenfalls mit wenigstens einer Oxo-, Formyl-, C₁-C₈-Acyl- oder C₆-C₁₀-Arylcarbonyl-Gruppe substituiert und unabhängig davon R² und R³ Ober eine Einfachbindung, Kohlenstoff-, Stickstoff-, Schwefel- und/oder Sauerstoffatome, unter Bildung eines 4 bis 8 Atome enthaltenden Rings miteinander verbunden sein können.

21. Verfahren nach irgendeinem der Ansprüche 16, 17 und 20, **dadurch gekennzeichnet, dass** die Ketone ausgewählt sind aus der Gruppe bestehend aus Pentanon, Hexanon, Heptanon, Octanon, 3-Penten-2-on, 5-Hexen-2-on, 4-Hexen-3-on, 5-Methyl-3-hexen-2.on, 5-Methyl-5-hexen-2-on, 6-Methyl-5-hepten-2-on, 3-Nonen-2-on, Phoron, Nerylaceton, Geranylaceton, Adamantylketon, Campher, Cyclohexenon, Benzochinon, Benzylaceton, Acetophenon, Valerophenon, 1,2-Diacetylbenzol, 4,4'-Diacetylbiphenyl und 1-Acetonaphthenon.

22. Verfahren nach Anspruch 16, **dadurch gekennzeichnet, dass** die primären Amine Verbindungen mit bis zu 25 Kohlenstoffatomen gemäß der Formel R⁴[-NH-R⁵]_{n"} sind,
wobei n" = 1,2 oder 3 ist
und R⁴ ausgewählt ist aus der Gruppe bestehend aus
- linearen oder verzweigten, gesättigten oder ungesättigten, aliphatischen oder alicyclischen Alkyl-Gruppen mit 1 bis 12, vorzugsweise 2 bis 8, besonders bevorzugt 2 bis 4 Kohlenstoffatomen;
- Aryl-, Aryl-C₁-C₄-Alkyl- und Aryl-C₂-C₆-Alkenyl-Gruppen mit 5 bis 12 Kohlenstoffatomen im Arylrest, die gegebenenfalls substituiert sein können mit C₁-C₆-Alkyl- und/oder C₂-C₆-Alkoxy-Gruppen; und
- Heteroaryl-Gruppen mit 3 bis 8 Kohlenstoffatomen und einem oder zwei Heteroatom(en) ausgewählt aus N, O und S, die substituiert sein können mit C₁-C₆-Alkyl- und/oder C₂-C₆-Alkoxy-Gruppen,
wobei die Alkyl-, Aryl- und Heteroaryl-Gruppen gegebenenfalls mit wenigstens einer Oxo-, Formyl-, C₁-C₆-Acyl- oder C₆-C₁₀-Arylcarbonyl-Gruppe substituiert sein können, während R⁶ ausgewählt ist aus Wasserstoff.

23. Verfahren nach irgendeinem der Ansprüche 16, und 22, **dadurch gekennzeichnet, dass** die Amine ausgewählt sind aus der Gruppe bestehend aus Butylamin, Amylamin, Hexylamin, Heptylamin, Octylamin, Nonylamin, Decylamin, Undecylamin, Dodecylamin, Tridecylamin, 1-Tetradecylamin, Isobutylamin, *tert*-Butylamin, 1-Methylbutylamin, 1-Ethylpropylamin, 2-Methylbutylamin, Isoamylamin, 1,2-Dimethylpropylamin, *tert*-Amylamin, 1,3-Dimethylbutylamin, 3,3-Dimethylbutylamin, 2-Aminoheptan, 3-Aminoheptan, Cyclopentylamin, Cyclohexylamin, Cyclohexanmethylamin, 3-Methoxypropylamin, 3-Ethoxypropylamin, 3-Butoxypropylamin, Tetrahydrofurfurylamin, Ethanolamin, 3-Amino-1-propanol, DL-2-Amino-1-propanol, DL-1-Amino-2-propanol, 4-Amino-l-butanol, 2-Amino-1-butanol, 2-Amino-2-methyl-propanol. 5-Amino-1-propanol, DL-2-Amino-1-pentanol, 6-Amino-1-hexanol, DL-2-Amino-1-hexanol, Aminocyclohexanol, Serinol, 1-Amino-l-deoxy-D-sorbitol, D-Gallosamin, D-Glucosamin, D-Mannosamin, Benzylamin, Phenylethylamin, 3-Phenyl-1-propylamin, 3,3-Diphenylpropylamin, 4-Phenylbutylamin, N-Phenylethylendiamin, 2-Amino-1-phenylethanol, 2-Methoxybenzylamin, 2-Methoxyphenethylamin und 2-Ethoxybenzylamin.

24. Verfahren gemäß irgendeinem der vorigen Ansprüche, **dadurch gekennzeichnet, dass** die reaktiven Komponenten (A) und (B) gleichzeitig oder unabhängig voneinander und nacheinander auf die Fasern appliziert werden.

25. Verfahren gemäß irgendeinem der vorigen Ansprüche, **dadurch gekennzeichnet, dass** die zu behandelnden Fasern ausgewählt sind aus menschlichen und tierischen Haaren, Wolle, Seide, Cellulosefasern, Kunstfasern, Fellen und Pelzen.

26. Haar, erhaltlich durch das Verfahren gemäß irgendeinem der Ansprüche 1 bis 25.

27. Zusammensetzung, umfassend
- wenigstens eine reaktive Komponente (A) mit einem Molekulargewicht ≤1000 Dalton, die wenigstens eine funktionelle Gruppe aufweist, ausgewählt aus primären Amino-Gruppen,
- wenigstens eine komplementäre reaktive Komponente (B) mit einem Molekulargewicht ≤1000 Dalton, die wenigstens eine funktionelle Gruppe aufweiset, ausgewählt aus Carbonyl-Gruppen,
wobei die Komponenten (A) und (B) miteinander unter Bildung einer Schiffschen Base reagieren, und wobei sich wenigstens eine der Komponenten (A) und (B) von einem Wirkstoff ableitet, der ausgewählt ist aus der Gruppe, bestehend aus Kohlenhydraten, Aminosäuren, Vitaminen und UV-Filtern, wobei die in den reaktiven Komponenten enthaltenen funktionellen Gruppen durch geeignete Derivatisierung In die Wirkstoffmoleknie eingeführt werden können.

28. Kit-of-parts umfassend Komponenten (A) und (B) der Zusammensetzung nach Anspruch 27 räumlich getrennt voneinander.

29. Verbindung der Formel VI

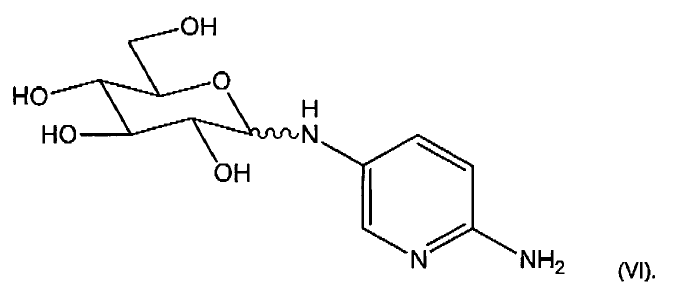

## Claims

1. A method for achieving a durable and positive influence on the volume, wet and dry combability, hold, moisture content, body, gloss, strength, tactility, electrostatic properties, elevated resistance to UV and IR radiation and exposure to heat of fibres, in particular of human and animal hair, by immobilising active ingredients on fibres, by treating them with a multicomponent system comprising
- at least one reactive component (A) with a molecular weight of ≤1000 dalton which comprises at least one functional group which is selected from primary amino groups,
- at least one complementary reactive component (B) with a molecular weight of ≤1000 dalton which comprises at least one functional group which is selected from carbonyl groups,
components (A) and (B) reacting with one another to form a Schiff base and at least one of components (A) and (B) being derived from an active ingredient which is selected from the group consisting of carbohydrates, amino acids, vitamins and UV filters, it being possible to introduce the functional groups present in the reactive components into the active ingredient molecule by suitable derivatisation.

2. A method according to claim 1, **characterised in that** the carbohydrate from which reactive components (A) and/or (B) are derived is selected from the group consisting of aldohexoses, aldopentoses, aldofuranoses, aldopyranoses, glycosamines of aldofuranoses, dimeric reducing aldopyranoses and the glycosamines formed therefrom, dimeric reducing aldofuranoses and the glycosamines formed therefrom, trimeric carbohydrates, glycuronic acids, aldonic acids or the lactones derived therefrom.

3. A method according to claim 2, **characterised in that** the carbohydrate from which reactive components (A) and/or (B) are derived is selected from the group consisting of allose, altrose, galactose, glucose, gulose, idose, mannose, talose, glucosamine, galactosamine, mannosamine, maltose, cellobiose, gentiobiose, lactose, lactosamine, melezitose, panose, erythrose, threose, ribose, arabinose, xylose, lyxose, glucuronic acid, glucono-1,5-lactone, 1',2-O-isopropylidene-D-isosaccharin lactone, xylonic acid γ-lactone, ribonic acid γ-lactone, gulonic acid γ-lactone, mannonic acid γ-lactone, erythronic acid γ-lactone, glucoheptanoic acid γ-lactone, glucooctanoic acid γ-lactone.

4. A method according to either of claim 2 and claim 3, **characterised in that** the pyranoses are derivatised in position 1 (anomeric centre), 2 and/or 6, the furanoses in positions 1 (anomeric centre), 2 or 5.

5. A method according to any one of the preceding claims, **characterised in that** the active ingredients are amino-derivatised using amines according to the following formula
H₂N-R-NH₂ (I)
wherein R is
- a saturated or unsaturated, branched or linear, aliphatic or alicyclic alkylene group containing 2 to 12 carbon atoms,
- an arylene group containing 5 to 10 carbons,
- a heteroarylene group with up to 2 nitrogen atoms containing 3 to 5 carbon atoms,
- a structural element of the following formula (II), 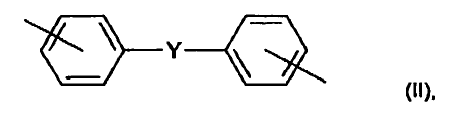 in which Y is selected from
- an aliphatic alkylene group containing 1 to 5 carbons,
- an alkenylene group containing 2 to 5 carbons,
- a sulfide group (-S-) or a disulfide group (-S-S-).

6. A method according to claim 5, **characterised in that** the amines used for amino-derivatisation of the active ingredients are selected from the group consisting of 1,2-diaminoethane, 1,2- or 1,3-diaminopropane, 1,2-, 1,3- or 1,4-diaminobutane, 1,5-diaminopentane, 2,2-dimethyl-1,3-diaminopropane, hexamethylenediamine, 1,7-diaminoheptane, 1,8-diaminooctane, trimethyl-1,6-diaminohexane, 1,9-diaminononane, 1,10-diaminodecane, 1,12-diaminododecane, 1,2-diaminocyclohexane, 1,4-diaminocyclohexane, 1,3-cyclohexane-bis(methylamine), 1,2-phenylenediamine, 1,3-phenylenediamine, 1,4-phenylenediamine, 4,4'-ethylenedianiline, 4,4'-methylenedianiline, 4,4'-diaminostilbene, 4,4'-thiodianiline, 4-aminophenyl disulfide, 2,6-diaminopyridine, 2,3-diaminopyridine, 3,4-diaminopyridine, 2,4-diaminopyrimidine, 4,5-diaminopyrimidine and 4,6-diaminopyrimidine.

7. A method according to claim 1, **characterised in that** reactive component (A) is an amino-derivatised carbohydrate according to formula III below or a hydrohalide derived therefrom

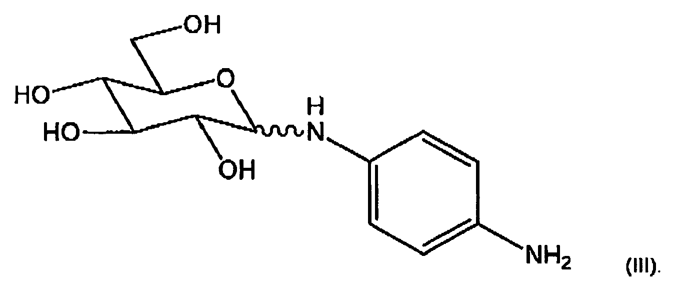

8. A method according to claim 1, **characterised in that** reactive component (A) is an amino-derivatised carbohydrate according to formula IV below or a hydrohalide derived therefrom

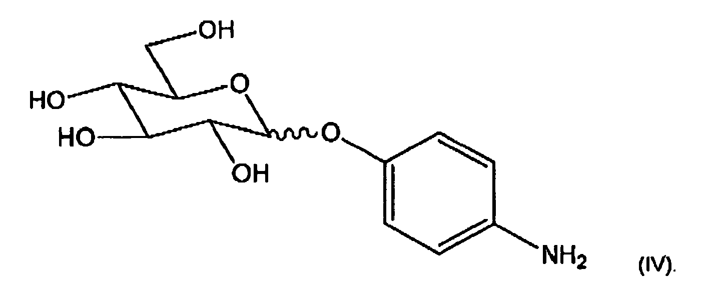

9. A method according to claim 1, **characterised in that** reactive component (A) is an amino-derivatised carbohydrate according to formula V below or a hydrohalide derived therefrom

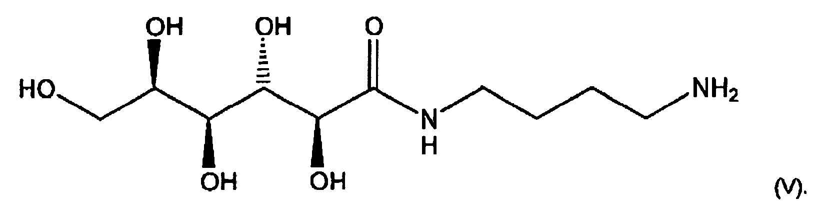

10. A method according to claim 1, **characterised in that** reactive component (A) is an amino-derivatised carbohydrate according to formula VI below or a hydrohalide derived therefrom

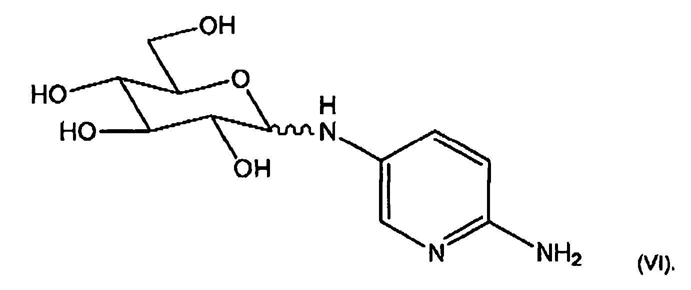

11. A method according to claim 1, **characterised in that** reactive component (A) is an amino-derivatised carbohydrate according to formula VII below or a hydrohalide derived therefrom

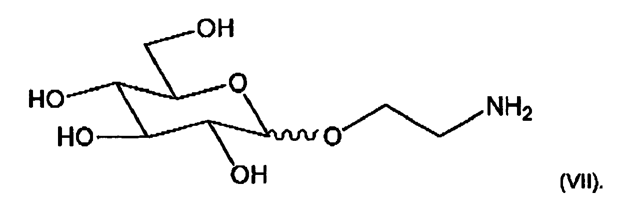

12. A method according to claim 1, **characterised in that** reactive component (B) is a carbonyl-derivatised carbohydrate according to formula VIII below

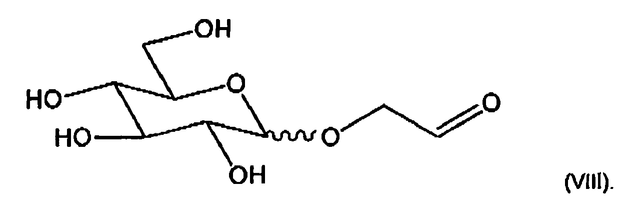

13. A method according to claim 1, **characterised in that** the amino acid from which reactive component (A) and/or (B) is derived is selected from the group consisting of glycine, alanine, valine, leucine, serine, threonine, cystine, cysteine, methionine, arginine, lysine, asparagine, aspartic acid, glutamine, glutamic acid, phenylalanine, tyrosine, proline, tryptophan and histidine.

14. A method according to claim 1, **characterised in that** the vitamin from which reactive components (A) and/or (B) are derived is selected from the group consisting of retinol, riboflavine, tocopherol, nicotinic acid and the derivatives thereof, pantothenic acid, biotin, folic acid, linoleic acid, arachidonic acid, ascorbic acid, pyridoxal and pyridoxamine.

15. A method according to claim 1, **characterised in that** the UV filter from which reactive components (A) and/or (B) are derived is selected from the group consisting of derivatives of cyanuric chloride, 2-hydroxy-4-methoxybenzophenone, 4-aminobenzoic acid, 3,3,5-trimethylcyclohexyl salicylate, 3-imidazol-4-ylacrylic acid, 4-aminobenzoic acid 1-glyceryl ester, salicylic acid 2-ethylhexyl ester and 4-hydroxy-4-methoxy-4-methylbenzophenone.

16. A method according to claim 1, **characterised in that** reactive component (A) is selected from primary amines and reactive component (B) is selected from aldehydes and ketones.

17. A method according to claim 16, **characterised in that** the aldehydes may contain up to 25 carbon atoms and the ketones up to 40 carbon atoms.

18. A method according to either of claim 16 and claim 17, **characterised in that** the aldehydes are compounds of the formula R¹[-CHO]ₙ, wherein n is 1, 2 or 3 and R¹ is selected from the group consisting of
- linear or branched, saturated or unsaturated, aliphatic or alicyclic alkyl groups with 1 to 12 carbon atoms;
- aryl, aryl-C₁-C₄-alkyl and aryl-C₂-C₆-alkenyl groups with 5 to 12 carbon atoms in the aryl residue, which may optionally be substituted with C₁-C₆ alkyl and/or C₂-C₆ alkoxy groups; and
- heteroaryl groups with 3 to 8 carbon atoms and one or two heteroatom(s) selected from N, O and S, which may be substituted with C₁-C₆ alkyl and/or C₂-C₆ alkoxy groups,
wherein the alkyl, aryl and heteroaryl groups may optionally be substituted with at least one oxo, formyl, C₁-C₈ acylcarbonyl or C₆-C₁₀ arylcarbonyl group.

19. A method according to any one of claims 16 to 18, **characterised in that** the aldehydes are selected from the group consisting of benzaldehyde, cinnamaldehyde, trans-stilbene carboxaldehyde, helicin, dihydroxycarboxyphenyl glycoside, diformylpiperazine, pyridine carboxaldehyde, pyrrole carboxaldehyde, pyridoxal, phthalic dialdehyde, citronellal and valeraldehyde.

20. A method according to either of claim 16 and claim 17, **characterised in that** the ketones are compounds of the formula R²-(C=O)-R³, wherein R² and R³ are mutually independently selected from the group consisting of
- linear or branched, saturated or unsaturated, aliphatic or alicyclic alkyl groups with 1 to 12 carbon atoms;
- aryl, aryl-C₁-C₄-alkyl and aryl-C₂-C₆-alkenyl groups with 5 to 12 carbon atoms in the aryl residue, which may optionally be substituted with C₁-C₆ alkyl and/or C₂-C₆ alkoxy groups; and
- heteroaryl groups with 3 to 8 carbon atoms and one or two heteroatom(s) selected from N, O and S, which may optionally be substituted with C₁-C₆ alkyl and/or C₂-C₆ alkoxy groups,
wherein the alkyl, aryl and heteroaryl groups may optionally be substituted with at least one oxo, formyl, C₁-C₈ acylcarbonyl or C₆-C₁₀ arylcarbonyl group and, independently thereof, R² and R³ may be joined to one another via a single bond, carbon, nitrogen, sulfur and/or oxygen atoms to form a ring containing 4 to 8 atoms.

21. A method according to any one of claims 16, 17 and 20, **characterised in that** the ketones are selected from the group consisting of pentanone, hexanone, heptanone, octanone, 3-penten-2-one, 5-hexen-2-one, 4-hexen-3-one, 5-methyl-3-hexen-2-one, 5-methyl-5-hexen-2-one, 6-methyl-5-hepten-2-one, 3-nonen-2-one, phorone, neryl acetone, geranyl acetone, adamantyl ketone, camphor, cyclohexenone, benzoquinone, benzyl acetone, acetophenone, valerophenone, 1,2-diacetylbenzene, 4,4'-diacetylbiphenyl and 1-acetonaphthenone.

22. A method according to claim 16, **characterised in that** the primary amines are compounds with up to 25 carbon atoms of the formula R⁴[-NH-R⁵]_{n"},
wherein n" is 1, 2 or 3
and R⁴ is selected from the group consisting of
- linear or branched, saturated or unsaturated, aliphatic or alicyclic alkyl groups with 1 to 12, preferably 2 to 8, particularly preferably 2 to 4 carbon atoms;
- aryl, aryl-C₁-C₄-alkyl and aryl-C₂-C₆-alkenyl groups with 5 to 12 carbon atoms in the aryl residue, which may optionally be substituted with C₁-C₆ alkyl and/or C₂-C₆ alkoxy groups; and
- heteroaryl groups with 3 to 8 carbon atoms and one or two heteroatom(s) selected from N, O and S, which may be substituted with C₁-C₆ alkyl and/or C₂-C₆ alkoxy groups,
wherein the alkyl, aryl and heteroaryl groups may optionally be substituted with at least one oxo, formyl, C₁-C₈ acylcarbonyl or C₆-C₁₀ arylcarbonyl group,
while R⁵ is selected from hydrogen.

23. A method according to either of claim 16 and claim 22, **characterised in that** the amines are selected from the group consisting of butylamine, amylamine, hexylamine, heptylamine, octylamine, nonylamine, decylamine, undecylamine, dodecylamine, tridecylamine, 1-tetradecylamine, isobutylamine, *tert*.-butylamine, 1-methylbutylamine, 1-ethylpropylamine, 2-methylbutylamine, isoamylamine, 1,2-dimethylpropylamine, *tert*.-amylamine, 1,3-dimethylbutylamine, 3,3-dimethylbutylamine, 2-aminoheptane, 3-aminoheptane, cyclopentylamine, cyclohexylamine, cyclohexanemethylamine, 3-methoxypropylamine, 3-ethoxypropylamine, 3-butoxypropylamine, tetrahydrofurfurylamine, ethanolamine, 3-amino-1-propanol, DL-2-amino-1-propanol, DL-1-amino-2-propanol, 4-amino-1-butanol, 2-amino-1-butanol, 2-amino-2-methyl-1-propanol, 5-amino-1-propanol, DL-2-amino-1-pentanol, 6-amino-1-hexanol, DL-2-amino-1-hexanol, aminocyclohexanol, serinol, 1-amino-1-deoxy-D-sorbitol, D-gulosamine, D-glucosamine, D-mannosamine, benzylamine, phenylethylamine, 3-phenyl-1-propylamine, 3,3-diphenylpropylamine, 4-phenylbutylamine, N-phenylethylenediamine, 2-amino-1-phenylethanol, 2-methoxybenzylamine, 2-methoxyphenethylamine and 2-ethoxybenzylamine.

24. A method according to any one of the preceding claims, **characterised in that** reactive components (A) and (B) are applied simultaneously or mutually independently and in succession onto the fibres.

25. A method according to any one of the preceding claims, **characterised in that** the fibres to be treated are selected from human and animal hair, wool, silk, cellulose fibres, synthetic fibres, hides and furs.

26. Hair obtainable by the method according to any one of claims 1 to 25.

27. A composition comprising
- at least one reactive component (A) with a molecular weight of ≤1000 dalton which comprises at least one functional group selected from primary amino groups,
- at least one complementary reactive component (B) with a molecular weight of ≤1000 dalton which comprises at least one functional group selected from carbonyl groups,
components (A) and (B) reacting with one another to form a Schiff base and at least one of components (A) and (B) being derived from an active ingredient which is selected from the group consisting of carbohydrates, amino acids, vitamins and UV filters, it being possible to introduce the functional groups present in the reactive components into the active ingredient molecule by suitable derivatisation.

28. A kit of parts comprising components (A) and (B) of the composition according to claim 27 spatially separate from one another.

29. A compound of the formula VI

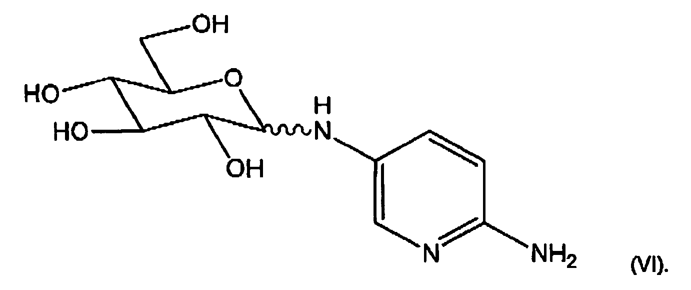

## Revendications

1. Procédé pour l'obtention d'une influence durable et positive sur le volume, sur le démêlage au mouiller et à sec, sur la tenue, sur l'humidité, sur le volume, sur le brillant, sur la résistance, sur la tactilité, sur les propriétés électrostatiques, sur une résistance élevée contre les expositions aux rayonnements UV et IR et contre l'influence de la chaleur de fibres, en particulier de cheveux humains et de poils d'animaux, par immobilisation de substances actives sur des fibres, via leur traitement avec un système à plusieurs composants, comprenant
- au moins un composant réactif (A) possédant un poids moléculaire ≤ 1000 Daltons qui présente au moins un groupe fonctionnel qui est choisi parmi des groupes amino primaires ;
- au moins un composant réactif complémentaire (B) possédant un poids moléculaire ≤ 1000 Daltons qui présente au moins un groupe fonctionnel qui est choisi parmi des groupes carbonyle ;
les composants (A) et (B) réagissant l'un avec l'autre en formant une base de Schiff, et au moins un des composants (A) et (B) dérivant d'une substance active qui est choisie parmi le groupe constitué par des hydrates de carbone, des acides aminés, des vitamines et des filtres UV, les groupes fonctionnels contenus dans les composants réactifs pouvant être introduits dans les molécules des substances actives par une dérivation appropriée.

2. Procédé selon la revendication 1, **caractérisé en ce que** l'hydrate de carbone, dont dérive(nt) le ou les composants réactifs (A) et/ou (B) est choisi parmi le groupe constitué par des aldohexoses, des aldopentoses, des aldofuranoses, des aldopyranoses, des glycosamines d' aldofuranoses, des aldopyranoses réducteurs dimères et des glycosamines formées à partir de ces derniers, des aldofuranoses réducteurs dimères et des glycosamines formées à partir de ces derniers, des hydrates de carbone trimères, des acides glycuroniques, des acides aldoniques ou des lactones qui en dérivent.

3. Procédé selon la revendication 2, **caractérisé en ce que** l'hydrate de carbone, dont dérive(nt) le ou les composants réactifs (A) et/ou (B) est choisi parmi le groupe constitué par l'allose, l'altrose, le galactose, le glucose, le gulose, l'idose, le mannose, le talose, la glucosamine, la galactosamine, la mannosamine, le maltose, le cellobiose, le gentiobiose, le lactose, la lactosamine, le mélézitose, le panose, l'érythrose, le thréose, le ribose, l'arabinose, le xylose, le lyxose, l'acide glucuronique, la gluono-1,5-lactone, la 1',2-O-isopropylidène-D-isosaccharine-lactone, la γ-lactone de l'acide xylonique, la γ-lactone de l'acide ribonique, la γ-lactone de l'acide gulonique, la γ-lactone de l'acide mannonique, la γ-lactone de l'acide érythronique, la γ-lactone de l'acide glucoheptanoïque, la γ-lactone de l'acide gluco-octanoïque.

4. Procédé selon l'une quelconque des revendications 2 et 3, **caractérisé en ce que** les pyranoses aux positions 1 (centre anomère), 2 et/ou 6, les furanoses aux positions 1 (centre anomère), 2 ou 5 sont dérivés.

5. Procédé selon l'une quelconque des revendications précédentes, **caractérisé en ce que**, pour la dérivation des substances actives sur le groupe amino, on utilise des amines répondant à la formule suivante
H₂N-R-NH₂ (I)
dans laquelle R représente
- un groupe alkylène aliphatique ou alicyclique, ramifié ou linéaire, saturé ou insaturé, contenant de 2 à 12 atomes de carbone ;
- un groupe arylène contenant de 5 à 10 atomes de carbone ;
- un groupe hétéroarylène comprenant jusqu'à 2 atomes d'azote contenant de 3 à 5 atomes de carbone ;
- un élément de structure répondant à la formule (II) ci-après 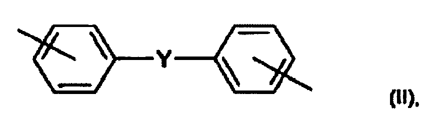 dans laquelle Y est choisi parmi
- un groupe alkylène aliphatique contenant de 1 à 5 atomes de carbone ;
- un groupe alkylène contenant de 2 à 5 atomes de carbone ;
- un groupe sulfure (-S-) ou un groupe disulfure (-S-S-).

6. Procédé selon la revendication 5, **caractérisé en ce que** les amines utilisées pour la dérivation des substances actives sur le groupe amino sont choisies parmi le groupe constitué par le 1,2-diaminoéthane, le 1,2-ou 1,3-diaminopropane, le 1,2-, 1,3- ou 1,4-diaminobutane, le 1,5-diaminopentane, le 2,2-diméthyl-1,3-diaminopropane, l'hexaméthylènediamine, le 1,7-diamino-heptane, le 1,8-diamino-octane, le triméthyl-1,6-diaminohexane, le 1,9-diaminononane, le 1,10-diaminodécane, le 1,12-diaminododécane, le 1,2-diaminocyclohexane, le 1,4-diaminocyclohexane, la 1,3-cyclohexane-bis(méthylamine), la 1,2-phénylènediamine, la 1,3-phénylènediamine, la 1,4-phénylènediamine, la 4,4'-éthylènedianiline, la 4,4'-méthylènedianiline, le 4,4'-diaminostilbène, la 4,4'-thiodianiline, le 4-aminophényldisulfure, la 2,6-diaminopyridine, la 2,3-diaminopyridine, la 3,4-diaminopyridine, la 2,4-diaminopyrimidine, la 4,5-diaminopyrimidine et la 4,6-diaminopyrimidine.

7. Procédé selon la revendication 1, **caractérisé en ce que** le composant réactif (A) représente un hydrate de carbone soumis à une dérivation sur le groupe amino, répondant à la formule III ci-après ou un hydrohalogénure qui en dérive

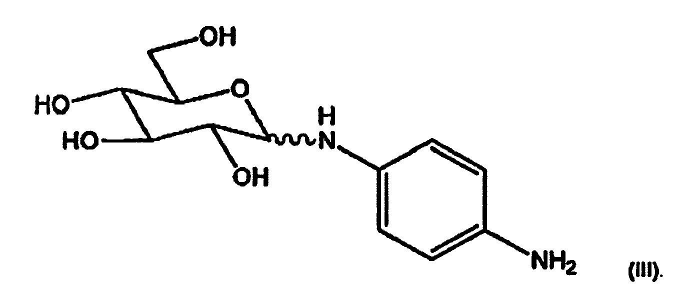

8. Procédé selon la revendication 1, **caractérisé en ce que** le composant réactif (A) représente un hydrate de carbone soumis à une dérivation sur le groupe amino, répondant à la formule IV ci-après ou un hydrohalogénure qui en dérive

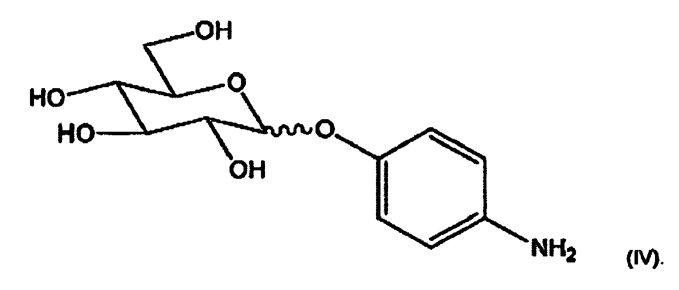

9. Procédé selon la revendication 1, **caractérisé en ce que** le composant réactif (A) représente un hydrate de carbone soumis à une dérivation sur le groupe amino, répondant à la formule V ci-après ou un hydrohalogénure qui en dérive

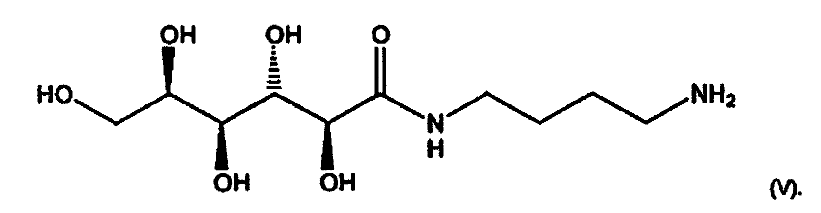

10. Procédé selon la revendication 1, **caractérisé en ce que** le composant réactif (A) représente un hydrate de carbone soumis à une dérivation sur le groupe amino, répondant à la formule VI ci-après ou un hydrohalogénure qui en dérive

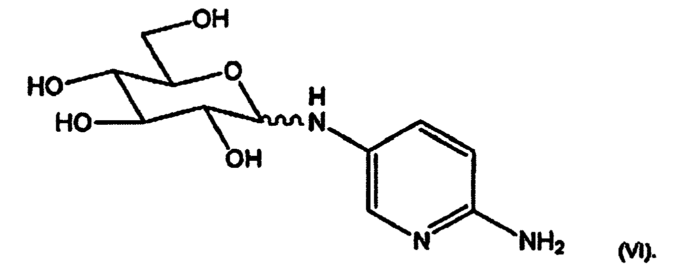

11. Procédé selon la revendication 1, **caractérisé en ce que** le composant réactif (A) représente un hydrate de carbone soumis à une dérivation sur le groupe amino, répondant à la formule VII ci-après ou un hydrohalogénure qui en dérive

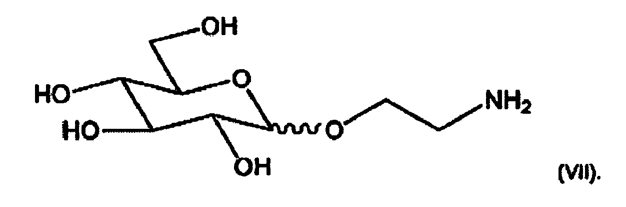

12. Procédé selon la revendication 1, **caractérisé en ce que** le composant réactif (B) représente un hydrate de carbone soumis à une dérivation sur le groupe carbonyle, répondant à la formule VIII ci-après

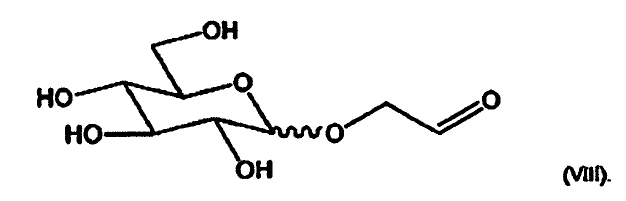

13. Procédé selon la revendication 1, **caractérisé en ce que** l'acide aminé, dont dérive(nt) le ou les composants réactifs (A) et/ou (B) est choisi parmi le groupe constitué par la glycine, l'alanine, la valine, la leucine, la sérine, la thréonine, la cystine, la cystéine, la méthionine, l'arginine, la lysine, l'asparagine, l'acide asparaginique, la glutamine, l'acide glutaminique, la phénylalanine, la tyrosine, la proline, le tryptophane et l'histidine.

14. Procédé selon la revendication 1, **caractérisé en ce que** la vitamine, dont dérive(nt) le ou les composants réactifs (A) et/ou (B) est choisie parmi le groupe constitué par le rétinol, la riboflavine, le tocophérol, l'acide nicotinique et ses dérivés, l'acide pantothénique, la biotine, l'acide folique, l'acide linoléique, l'acide arachidonique, l'acide ascorbique, le pyridoxal et la pyridoxamine.

15. Procédé selon la revendication 1, **caractérisé en ce que** le filtre UV, dont dérive(nt) le ou les composants réactifs (A) et/ou (B) est choisi parmi le groupe constitué par des dérivés du chlorure de cyanure, la 2-hydroxy-4-méthoxy-benzophénone, l'acide 4-aminobenzoïque, le salicylate de 3,3,5-triméthylcyclohexyle, l'acide 3-imidazol-4-yl-acrylique, l'ester 1-glycérylique de l'acide 4-amino-benzoïque, l'ester 2-éthyl-hexylique de l'acide salicylique et la 4-hydroxy-4-méthoxy-4-méthylbenzophénone.

16. Procédé selon la revendication 1, **caractérisé en ce que** le composant réactif (A) est choisi parmi des amines primaires et le composant réactif (B) est choisi parmi des aldéhydes et des cétones.

17. Procédé selon la revendication 16, **caractérisé en ce que** les aldéhydes peuvent contenir jusqu'à 25 atomes de carbone et les cétones peuvent contenir jusqu'à 40 atomes de carbone.

18. Procédé selon l'une quelconque des revendications 16 et 17, **caractérisé en ce que** les aldéhydes représentent des composés répondant à la formule R¹[-CHO]ₙ, dans laquelle n = 1, 2 ou 3 et R¹ est choisi parmi le groupe constitué par
- des groupes alkyle aliphatiques ou alicycliques, saturés ou insaturés, linéaires ou ramifiés, contenant de 1 à 12 atomes de carbone ;
- des groupes aryle, des groupes arylalkyle en C₁-C₄ et des groupes arylalcényle en C₂-C₆ contenant de 5 à 12 atomes de carbone dans le radical aryle, qui peuvent être substitués le cas échéant avec des groupes alkyle en C₁-C₆ et/ou des groupes alcoxy en C₂-C₆ ; et
- des groupes hétéroaryle contenant de 3 à 8 atomes de carbone et un ou deux hétéroatome(s) choisi(s) parmi un atome d'azote, un atome d'oxygène et un atome de soufre, qui peuvent être substitués avec des groupes alkyle en C₁-C₆ et/ou des groupes alcoxy en C₂-C₆;
les groupes alkyle, les groupes aryle et les groupes hétéroaryle pouvant être substitués, le cas échéant, avec au moins un groupe oxo, un groupe formyle, un groupe acyl(en C₁-C₈)carbonyle ou un groupe alkyl(en C₆-C₁₀)carbonyle.

19. Procédé selon l'une quelconque des revendications 16 à 18, **caractérisé en ce que** les aldéhydes sont choisis parmi le groupe constitué par le benzaldéhyde, le cinnamaldéhyde, le trans-stilbènecarboxaldéhyde, l'hélicine, le dihydroxycarboxyphénylglycoside, la diformylpipérazine, le pyridinecarboxaldéhyde, le pyrrolcarboxaldéhyde, le pyridoxal, le phtaldialdéhyde, le citronnellal et le valéraldéhyde.

20. Procédé selon l'une quelconque des revendications 16 et 17, **caractérisé en ce que** les cétones sont des composés répondant à la formule R²-(C=O)-R³, dans laquelle R² et R³ sont choisis, indépendamment l'un de l'autre, parmi le groupe constitué par
- des groupes alkyle aliphatiques ou alicycliques, saturés ou insaturés, linéaires ou ramifiés, contenant de 1 à 12 atomes de carbone ;
- des groupes aryle, des groupes arylalkyle en C₁-C₄ et des groupes arylalcényle en C₂-C₆ contenant de 5 à 12 atomes de carbone dans le radical aryle, qui peuvent être substitués le cas échéant avec des groupes alkyle en C₁-C₆ et/ou des groupes alcoxy en C₂-C₆ ; et
- des groupes hétéroaryle contenant de 3 à 8 atomes de carbone et un ou deux hétéroatome(s) choisi(s) parmi un atome d'azote, un atome d'oxygène et un atome de soufre, qui peuvent être substitués, le cas échéant, avec des groupes alkyle en C₁-C₆ et/ou des groupes alcoxy en C₂-C₆;
les groupes alkyle, les groupes aryle et les groupes hétéroaryle pouvant être substitués, le cas échéant, avec au moins un groupe oxo, un groupe formyle, un groupe acyl(en C₁-C₈)carbonyle ou un groupe alkyl(en C₆-C₁₀)carbonyle et, indépendamment de cela, R² et R³ pouvant être reliés l'un à l'autre via une liaison simple, des atomes de carbone, des atomes d'azote, des atomes de soufre et/ou des atomes d'oxygène, en formant un noyau contenant de 4 à 8 atomes.

21. Procédé selon l'une quelconque des revendications 16, 17 et 20, **caractérisé en ce que** les cétones sont choisis parmi le groupe constitué par la pentanone, l'hexanone, l'heptanone, l'octanone, la 3-pentén-2-one, la 5-hexén-2-one, la 4-hexén-3-one, la 5-méthyl-3-hexén-2-one, la 5-méthyl-5-hexén-2-one, la 6-méthyl-5-heptén-2-one, la 3-nonén-2-one, la phorone, la nérylacétone, la géranylacétone, l'adamantylcétone, le camphre, la cyclohexénone, la benzoquinone, la benzylacétone, l'acétophénone, la valérophénone, le 1,2-diacétylbenzène, le 4,4'-diacétylbiphényle et la 1-acétonaphténone.

22. Procédé selon la revendication 16, **caractérisé en ce que** les amines primaires représentent des composés contenant jusqu'à 25 atomes de carbone, répondant à la formule R⁴(-NH-R⁵]_{n"},
dans laquelle n" = 1, 2 ou 3,
et R⁴ est choisi parmi le groupe constitué par
- des groupes alkyle aliphatiques ou alicycliques, saturés ou insaturés, linéaires ou ramifiés, contenant de 1 à 12, de préférence de 2 à 8, de manière particulièrement préférée de 2 à 4 atomes de carbone
- des groupes aryle, des groupes arylalkyle en C₁-C₄ et des groupes arylalcényle en C₂-C₆ contenant de 5 à 12 atomes de carbone dans le radical aryle, qui peuvent être substitués le cas échéant avec des groupes alkyle en C₁-C₆ et/ou des groupes alcoxy en C₂-C₆ ; et
- des groupes hétéroaryle contenant de 3 à 8 atomes de carbone et un ou deux hétéroatome(s) choisi(s) parmi un atome d'azote, un atome d'oxygène et un atome de soufre, qui peuvent être substitués avec des groupes alkyle en C₁-C₆ et/ou des groupes alcoxy en C₂-C₆ ;
les groupes alkyle, les groupes aryle et les groupes hétéroaryle pouvant être substitués, le cas échéant, avec au moins un groupe oxo, un groupe formyle, un groupe acyl(en C₁-C₈)carbonyle ou un groupe aryl(en C₆-C₁₀)carbonyle,
tandis que R⁵ est choisi parmi un atome d'hydrogène.

23. Procédé selon l'une quelconque des revendications 16 et 22, **caractérisé en ce que** les amines sont choisies parmi le groupe constitué par la butylamine, l'amylamine, l'hexylamine, l'heptylamine, l'octylamine, la nonylamine, la décylamine, la undécylamine, la dodécylamine, la tridécylamine, la 1-tétradécylamine, l'isobutylamine, la tert-butylamine, la 1-méthylbutylamine, la 1-éthylpropylamine, la 2-méthylbutylamine, l'isoamylamine, la 1,2-diméthylpropylamine, la tert-amylamine, la 1,3-diméthylbutylamine, la 3,3-diméthylbutylamine, le 2-aminoheptane, le 3-aminoheptane, la cyclopentylamine, la cyclohexylamine, la cyclohexaneméthylamine, la 3-méthoxypropylamine, la 3-éthoxypropylamine, la 3-butoxypropylamine, la tétrahydrofurfurylamine, l'éthanolamine, le 3-amino-1-propanol, le DL-2-amino-1-propanol, le DL-1-amino-2-propanol, le 4-amino-1-butanol, le 2-amino-1-butanol, le 2-amino-2-méthyl-1-propanol, le 5-amino-1-propanol, le DL-2-amino-1-pentanol, le 6-amino-1-hexanol, le DL-2-amino-1-hexanol, l'aminocyclohexanol, le sérinol, le 1-amino-1-désoxy-D-sorbitol, la D-gallosamine, la D-glucosamine, la D-mannosamine, la benzylamine, la phényléthylamine, la 3-phényl-1-propylamine, la 3,3-diphénylpropylamine, la 4-phényl-butylamine, la N-phényléthylènediamine, le 2-amino-1-phényléthanol,la 2-méthoxybenzylamine, la 2-méthoxyphénéthylamine et la 2-éthoxybenzylamine.

24. Procédé selon l'une quelconque des revendications précédentes, **caractérisé en ce que** les composants réactifs (A) et (B) peuvent être appliqués sur les fibres de manière simultanée ou indépendamment l'un de l'autre et l'un après l'autre.

25. Procédé selon l'une quelconque des revendications précédentes, **caractérisé en ce que** les fibres à traiter sont choisies parmi des cheveux humains et des poils d'animaux, de la laine, de la soie, des fibres de cellulose, des fibres synthétiques, des peaux et des fourrures.

26. Cheveu, que l'on obtient via le procédé selon l'une quelconque des revendications 1 à 25.

27. Composition, comprenant
- au moins un composant réactif (A) possédant un poids moléculaire ≤ 1000 Daltons qui présente au moins un groupe fonctionnel choisi parmi des groupes amino primaires ;
- au moins un composant réactif complémentaire (B) possédant un poids moléculaire ≤ 1000 Daltons qui présente au moins un groupe fonctionnel choisi parmi des groupes carbonyle ;
les composants (A) et (B) réagissant l'un avec l'autre en formant une base de Schiff, et au moins un des composants (A) et (B) dérivant d'une substance active qui est choisie parmi le groupe constitué par des hydrates de carbone, des acides aminés, des vitamines et des filtres UV, les groupes fonctionnels contenus dans les composants réactifs pouvant être introduits dans les molécules des substances actives par une dérivation appropriée.

28. Nécessaire comprenant les composants (A) et (B) de la composition selon la revendication 27, séparés les uns des autres dans l'espace.

29. Composé répondant à la formule VI
